(19) Europäisches Patentamt
European Patent Office
Office européen des brevets

(11) EP 2 221 038 A1

(12) **DEMANDE DE BREVET EUROPEEN**

(43) Date de publication:
**25.08.2010 Bulletin 2010/34**

(21) Numéro de dépôt: **10154374.2**

(22) Date de dépôt: **23.02.2010**

(51) Int Cl.:
***A61K 8/02*** *(2006.01)* ***A61K 8/49*** *(2006.01)*
***A61Q 1/00*** *(2006.01)* ***A61Q 3/02*** *(2006.01)*
***A61Q 5/00*** *(2006.01)*

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **23.02.2009 FR 0900825**
**12.03.2009 US 159448 P**

(71) Demandeur: **L'Oréal**
**75008 Paris (FR)**

(72) Inventeurs:
- **Giron, Franck**
**77164, Ferrieres en Brie (FR)**
- **Samain, Henri**
**91570, Bievres (FR)**

(74) Mandataire: **Tanty, François**
**Cabinet Nony**
**3, rue de Penthièvre**
**75008 Paris (FR)**

(54) **Procédé de maquillage avec application d'une couche de base et ensemble pour la mise en oeuvre d'un tel procédé**

(57) La présente invention concerne un procédé de photomaquillage des matières kératiniques humaines, dans lequel :
a) on applique sur les matières kératiniques une couche de base d'une première composition contenant au moins un agent optique capable de former écran à une longueur d'onde λ au moins temporairement,
b) on applique sur la couche de base une deuxième composition photorévélable thermiquement stable révélable par exposition à un rayonnement au moins de longueur d'onde λ.

EP 2 221 038 A1

## Description

La présente invention est relative au maquillage de la peau et autres matières kératiniques humaines, par exemple les lèvres, les ongles ou cheveux.

## Arrière-plan

Le maquillage se réalise habituellement par l'emploi de matière colorée que l'on dépose sur le corps ou le visage.
Le résultat final dépend non seulement de la qualité des produits utilisés, en particulier des ingrédients et des techniques de formulation mises en oeuvre, mais aussi de la dextérité de l'utilisateur.
Dans l'espoir de gagner en dextérité et ainsi améliorer le résultat du maquillage, certains utilisateurs suivent des formations. D'autres ne le font pas, se considérant peu aptes pour cela ou n'ayant pas de temps à y consacrer.
On a découvert qu'il est possible d'obtenir des résultats de maquillage satisfaisants grâce au photomaquillage. La précision du rendu dépasse ce que les utilisateurs obtiennent habituellement avec le maquillage conventionnel, et ce sans nécessiter de dextérité particulière, ni de formation.
De plus, le photomaquillage peut permettre l'obtention de résultats coloriels qui sortent de l'acception classique du maquillage. Cela peut être tout motif imitant un motif conventionnel de maquillage ou un texte, un logo, ...
Le photomaquillage repose sur l'utilisation d'au moins une composition photorévélable thermiquement stable capable d'être révélée par un rayonnement lumineux, par exemple un rayonnement UV, et de conserver un changement d'aspect lié à l'irradiation pendant au moins une heure.
Pour réaliser le photomaquillage, au moins une composition photorévélable thermiquement stable est déposée sur la zone à traiter sous la forme d'au moins une couche.
Au moment où la composition photorévélable thermiquement stable est appliquée, celle-ci est à l'état non révélé et peut être colorée ou incolore, selon les ingrédients utilisés.
L'irradiation de la couche de composition photorévélable thermiquement stable peut se faire de façon sélective, en l'irradiant de manière non uniforme. Ainsi, certaines régions peuvent ne pas être révélées et d'autres le sont et/ou des régions sont révélées à des degrés divers, conduisant à des intensités de couleur différentes.
L'énergie lumineuse utilisée reste relativement faible, et n'entraîne pas de bronzage de la peau.
Un procédé de photomaquillage est décrit dans le brevet EP 938 887 qui est incorporé par référence et met en oeuvre des agents photorévélables thermiquement stables que l'on applique sur la peau. Ce brevet décrit un agent photorévélable choisi parmi les diaryléthènes et les fulgides.
La demande US 2007/0038270 A1 divulgue divers procédés dans lesquels une composition photosensible est déposée selon un motif sur la peau ou exposée à une lumière correspondant à un motif à réaliser.
Il est requis, comme tout revêtement de maquillage, que le photomaquillage puisse être éliminé. Cette condition est importante et presqu'obligatoire car les utilisateurs de maquillage aiment se démaquiller pour retrouver la peau non maquillée, notamment le soir. De plus, les utilisateurs apprécient de pouvoir réappliquer un maquillage sur la peau, différent du premier. Si des traces du premier se maintenaient, la réalisation du second serait rendue problématique.
Un problème qui peut survenir avec le photomaquillage est un phénomène de tachage de la peau.
En effet, au cours du démaquillage, l'utilisateur peut laisser des traces de composition photorévélable thermiquement stable non révélée. Or, ces traces peuvent être amenées à se révéler par la suite, par exemple après quelques heures passées à la lumière ambiante. A ce moment, il peut être difficile pour l'utilisateur de procéder à un complément de démaquillage.

## Résumé

L'invention a pour objet, selon un premier de ses aspects, un procédé de photomaquillage des matières kératiniques humaines, dans lequel :

a) on applique sur les matières kératiniques une couche de base d'une première composition photoprotectrice contenant au moins un agent optique capable de former écran à une longueur d'onde $\lambda$ au moins temporairement, notamment une longueur d'onde appartenant à la plage à la plage 320 à 440 nm,
b) on applique sur la couche de base une deuxième composition photorévélable thermiquement stable révélable par exposition à un rayonnement au moins de longueur d'onde $\lambda$.

Par « former écran à une longueur d'onde $\lambda$ », il faut comprendre que l'agent optique atténue d'au moins un facteur d'atténuation de 2 le rayonnement de longueur d'onde $\lambda$, la mesure étant effectuée par le biais d'un appareil apte à mesurer le spectre d'absorption en restreignant la lumière d'irradiation à une zone de longueur d'onde $\lambda \pm 10$

nm. Le rapport $F_{\lambda-10,\lambda+10} = \dfrac{\int_{\lambda-10}^{\lambda+10} I(\lambda)d\lambda}{\int_{\lambda-10}^{\lambda+10} I(\lambda)T(\lambda)d\lambda}$ avec *I*(λ) et *T*(λ) tels que définis plus loin, donne le facteur d'atténuation à la longueur d'onde λ, *T*(λ) étant mesuré comme décrit plus loin.

L'invention permet de réduire le risque de tachage de la peau en rendant plus difficile la migration du ou des agents photorévélables de la composition photorévélable thermiquement stable vers les matières kératiniques sous-jacentes et leur révélation.

Cette migration peut être ralentie davantage, voire empêchée, lorsque les première et deuxième compositions sont non miscibles l'une dans l'autre, l'une des compositions étant par exemple aqueuse et l'autre non aqueuse ou inversement.

Ainsi, on peut choisir des ingrédients (solvants, adhésifs...) pour la deuxième composition qui ne sont pas solvants de la composition photorévélable thermiquement stable et inversement. On choisit par exemple un solvant organique, par exemple parmi les alcools ou les cétones, notamment l'éthanol ou l'acétone, l'acétate d'alkyle, les huiles carbonées, notamment l'isododécane, les silicones volatiles, pour la première composition photoprotectrice et un solvant aqueux ou hydroalcoolique pour la seconde composition photorévélable thermiquement stable, ou inversement.

On peut aussi choisir deux solvants organiques ou deux solvants aqueux pour les deux compositions, de telle sorte qu'au séchage une transformation ait lieu. Par exemple, on emploie une première composition contenant un latex. Ce dernier va, au séchage, coalescer et rendre la couche inerte face à l'application de la composition photorévélable thermiquement stable. Ainsi, la couche de base peut être appliquée plus ou moins longtemps avant le maquillage photorévélable, par exemple plus de 15 minutes avant, ce qui peut avoir pour effet de laisser à la couche de base le temps de sécher et de rendre celle-ci peu ou pas soluble dans la couche appliquée dessus, comme mentionné plus haut. De plus, en séchant, la couche de base peut former éventuellement une surface relativement lisse, facilitant l'application d'une couche d'épaisseur homogène de composition photorévélable thermiquement stable. La peau étant lisse, la seconde couche peut être plus fine et l'on évite des inhomogénéités d'épaisseur susceptibles de donner des effets visuels inesthétiques après révélation.

La couche de base peut être formée sur une surface plus étendue que la composition photorévélable thermiquement stable. Cela facilite l'application de la composition photorévélable thermiquement stable, car l'utilisateur n'a plus à se soucier de faire correspondre précisément les contours d'application des deux compositions.

La longueur d'onde λ peut appartenir au spectre UV (280 nm - 400 nm) ou proche UV (400 nm - 440 nm).

L'agent optique peut être un filtre solaire non photostable, d'indice de photostabilité inférieur ou égal à 80 %.

Lorsque la composition photorévélable thermiquement stable est révélable par exposition à un rayonnement UV, alors préférentiellement l'agent optique est un filtre solaire non photostable, d'indice de photostabilité inférieur ou égal à 80 %.

Un avantage qui en résulte est qu'en cas d'application de la couche de base sur la peau, l'utilisateur n'est pas complètement empêché de bronzer, même si la couche de base a été appliquée sur une étendue dépassant largement la composition photorévélable thermiquement stable. La couche de base peut perdre lors de l'exposition au soleil sa capacité de filtration des UV, ce qui permet à l'utilisateur de bronzer progressivement, au moins dans la zone non recouverte par la composition photorévélable thermiquement stable. Si le filtre solaire était photostable, l'utilisateur pourrait craindre d'appliquer de façon trop étendue la couche de base, sous peine de laisser une trace éventuelle sur son bronzage.

La composition photorévélable thermiquement stable peut, par exemple, être révélée à l'aide d'un imageur matriciel adressable.

Le cas échéant, au moins une couche intermédiaire peut être appliquée sur la couche de base de façon à se situer entre la couche de composition photorévélable thermiquement stable et la couche de base.

Cette couche intermédiaire peut avoir pour effet d'améliorer la tenue de la couche de composition photorévélable thermiquement stable sur la couche de base ou au contraire d'en faciliter l'enlèvement, lors du démaquillage par exemple. La couche intermédiaire peut être une couche d'un polymère ou de cire.

La couche intermédiaire peut notamment ne pas avoir de fonction de filtration de la longueur d'onde λ de révélation de la composition photorévélable thermiquement stable.

On peut aussi appliquer une couche d'une autre composition sous la couche de base pour faciliter l'accrochage de celle-ci sur les matières kératiniques par exemple. Ainsi, la couche de base peut ne pas être directement au contact de la peau. En variante, la couche de base est appliquée à même la peau ou autres matières kératiniques.

L'invention a encore pour objet, selon un autre de ses aspects, un ensemble comportant, au sein d'un même conditionnement :

- une première composition à appliquer sur les matières kératiniques, pour former une couche de base, contenant au moins un agent optique capable de former écran à au moins une longueur d'onde λ appartenant à l'intervalle allant de 280 nm à 440 nm, mieux de 320 nm à 440 nm, la première composition comportant un filtre solaire non photostable, d'indice de photostabilité inférieur ou égal à 80 %,
- une deuxième composition, photorévélable thermiquement stable, à appliquer sur la couche de base.

Le filtre solaire non photostable peut présenter initialement un pouvoir filtrant F vis-à-vis du rayonnement UV solaire supérieur ou égal à 2, mieux 5 ou 10.

Pour mesurer la photostabilité d'un filtre solaire, on dilue celui-ci dans un solvant $C_{12}$-$C_{15}$ alkyle benzoate de marque FINSOLV®. Le filtre parsol 1789 est un exemple de filtre non photostable, d'indice de photostabilité de l'ordre de 30 %, défini comme étant le rapport entre le pouvoir filtrant après une heure d'exposition à un rayonnement UVA produit par un irradiateur de marque SUNTEST sur le pouvoir filtrant initial.

L'ensemble peut comporter un irradiateur capable d'émettre sélectivement dans l'UV et/ou le visible, comme détaillé plus loin.

## Composition photorévélable thermiquement stable

Le photomaquillage est réalisé, conformément à l'invention, grâce à une composition photorévélable thermiquement stable adaptée.

La composition photorévélable thermiquement stable selon l'invention contient un ou plusieurs agents photorévélables thermiquement stables convenant à la réalisation d'un photomaquillage,c'est-à-dire qu'ils changent d'aspect sous l'effet d'un rayonnement lumineux.

Le ou les agents photorévélables thermiquement stables utilisés dans l'invention peuvent être des agents photochromes thermiquement stables ou des agents photorévélables irreversibles, c'est-à-dire qu'une fois le changement d'aspect obtenu, celui-ci reste permanent.

Selon le ou les agents photorévélables thermiquement stables utilisés, le photomaquillage peut se réaliser en révélant progressivement ce ou ces agents photorévélables thermiquement stables, par exposition à un rayonnement adapté, par exemple UV et/ou proche UV, ou en partant d'une composition photorévélable thermiquement stables comportant un ou plusieurs agents photorévélables thermiquement stables à l'état déjà révélé que l'on amène dans un état non révélé en appliquant un rayonnement adapté, par exemple visible hors proche UV.

Le photomaquillage peut mettre en oeuvre à la fois, par exemple successivement ou alternativement, une révélation d'un ou plusieurs agents photorévélables thermiquement stables et un effacement d'un ou plusieurs agents photorévélables thermiquement stables, de façon à obtenir précisément le résultat de maquillage recherché.

La composition photorévélable thermiquement stable peut être contenue, avec le ou les agents photorévélables thermiquement stable à l'état non révélé, dans un dispositif de conditionnement, avant l'application sur les matières kératiniques. Dans ce cas, la composition photorévélable thermiquement stable peut être appliquée sur les matières kératiniques avec le ou les agents photorévélables thermiquement stables à l'état non révélé, puis le rayonnement permettant de faire passer ce ou ces agents photochromes à l'état révélé est appliqué.

En variante, la composition photochromique est appliquée sur les matières kératiniques avec le ou les agents photorévélables thermiquement stables à l'état non révélé, puis ceux-ci sont amenés dans un état révélé, et un rayonnement est ensuite appliqué de manière sélective pour faire passer le ou agents photorévélables thermiquement stables, par exemple de façon localisée, à l'état non révélé, de façon à créer un ou plusieurs motifs par exemple et/ou obtenir la couleur recherchée.

Dans une autre variante, la composition photorévélable thermiquement stable est contenue dans le dispositif de conditionnement avec des agents photorévélables thermiquement stables à l'état révélé. La composition photorévélable thermiquement stable est alors appliquée avec le ou les agents photorévélables thermiquement stables à l'état révélé, et ceux-ci sont amenés dans un état non révélé de façon sélective, de façon à former un ou plusieurs motifs et/ou obtenir la couleur recherchée.

Lorsque l'on cherche à utiliser une composition photorévélable thermiquement stable dont le ou les agents photorévélables thermiquement stables sont à l'état déjà révélé lorsque la composition est appliquée sur les matières kératiniques, on peut éventuellement utiliser un dispositif de conditionnement comportant une source lumineuse permettant d'exposer la composition photorévélable thermiquement stables, par exemple au sein de l'enceinte du récipient la contenant ou au niveau d'un orifice de distribution ou d'un organe d'application, à un rayonnement lumineux de longueur d'onde adaptée à révéler le ou les agents photorévélables thermiquement stables.

La composition photorévélable thermiquement stable peut comporter un agent photorévélable thermiquement stable permettant par exemple de générer une couleur à l'état révélé, et par exemple incolore à l'état non révélé, ou un mélange d'agents photorévélables thermiquement stables produisant à l'état révélé des couleurs respectives différentes ayant, à l'état non révélé, une autre couleur ou étant incolores.

On peut par exemple utiliser une composition photorévélable thermiquement stable comportant un mélange d'agents photorévélables thermiquement stables respectivement jaune, bleu et magenta, avec par exemple une proportion plus importante d'agent photorévélable thermiquement stable dont la couleur est jaune à l'état révélé, les proportions étant par exemple choisies pour obtenir, lorsque tous les agents photorévélables thermiquement stables sont à l'état révélé, une teinte proche de celle de la peau. Dans un exemple de mise en oeuvre de l'invention, on utilise ainsi un mélange d'agents photorévélables thermiquement stables respectivement jaune, magenta et bleu, dans des proportions relatives de 50 %, 35 % et 15 % environ.

Lorsque la composition photorévélable thermiquement stable comporte plusieurs agents photorévélables thermiquement stables, on peut utiliser des agents photorévélables thermiquement stables qui sont révélables par exposition à des rayonnements de longueurs d'onde dominantes respectives différentes, de façon à ce qu'en choisissant la longueur d'onde du rayonnement à laquelle la composition photorévélable thermiquement stable est exposée, on puisse révéler plutôt une couleur qu'une autre. On peut aussi utiliser des agents photorévélables thermiquement stables au sein d'une composition photorévélable thermiquement stable qui sont effaçables lorsqu'exposés à des longueurs d'ondes dominantes respectives, ce qui permet en choisissant les caractéristiques de la lumière utilisée pour effacer la composition photorévélable thermiquement stable, d'effacer sélectivement une couleur donnée plutôt qu'une autre.

Mesure de la stabilité thermique d'un agent photorévélable thermiquement stable.

Le test pour déterminer si un agent photorévélable est thermiquement stable est le suivant. On soumet l'agent à tester, initialement de couleur $E_i$ à l'état non révélé, à une irradiation par un rayonnement UV pendant 1 minute à $1J/cm^2$, puis on détermine sa couleur finale $E_f$ à l'aide d'un spectrocolorimètre, par exemple celui de marque MINOLTA CM 2002 (d/8, SCI, D65, 2° observateur); on obtient un écart de couleur $\Delta E_{i,f} = \sqrt{(a_f - a_i)^2 + (b_f - b_i)^2 + (L_f - L_i)^2}$ dans l'espace CIE Lab, qui correspond au maximum de révélation. On laisse ensuite ledit composé dans l'obscurité totale pendant 60 minutes à 25°C, puis on détermine à nouveau sa couleur $E_r$ selon la méthode ci-dessus. Lorsque la nouvelle valeur de $\Delta E_{i,r}$ est au moins égale à 50% de la valeur de $\Delta E_{i,f}$ correspondant au maximum de révélation, on considère que le composé est thermiquement stable. De préférence, l'agent photorévélable thermiquement stable est choisi pour qu'une fois révélé, le maquillage obtenu puisse être conservé visuellement plus d'une heure, mieux plus de quatre heures.

La composition photorévélable thermiquement stable peut être dépourvue de composés photochromes thermiquement réversibles, tels que l'oxyde de titane dopé, les spiropyrannes, les spirooxazines ou les chromènes, sauf si certaines formes de ces composés entrent dans la définition d'agents photorévélables thermiquement stables.

Le ou les agents photochromes photorévélables thermiquement stables selon l'invention sont avantageusement tels que sous une première irradiation $I_1$, ce ou ces agents se révèlent en se colorant, en partant d'un état sensiblement incolore, ou peu coloré, et sous une deuxième irradiation $I_2$ différente de la première, ce ou ces agents redeviennent sensiblement incolores ou peu colorés. L'irradiation $I_1$ est dans des exemples de mise en oeuvre de l'invention une irradiation UV (290 nm à 400 nm), notamment UVA (320 à 400 nm) et/ou UVB, mieux dans le proche UV (400 à 440 nm), tandis que l'irradiation $I_2$ est une irradiation dans le visible, par exemple de la lumière blanche.

Agents photochromes thermiquement stables

Parmi les agents photochromes utilisables, les composés qui appartiennent à la famille des diaryléthènes et ceux qui appartiennent à la famille des fulgides sont préférés, cette liste n'étant toutefois pas limitative. L'homme du métier pourra se référer au brevet EP 938 887 qui décrit des exemples d'agents photochromes thermiquement stables.

Les diaryléthènes peuvent être représentés par la formule (I) suivante :

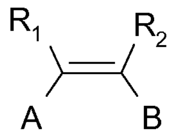

dans laquelle les radicaux R1 et R2 sont toujours en relation "cis" par rapport à la double liaison.

Ces radicaux R1 et R2 peuvent être, indépendamment l'un de l'autre, choisis parmi les radicaux alkyles en

C1-C16, éventuellement fluorés ou perfluorés, ou nitriles.

On peut notamment citer les composés de formule suivante :

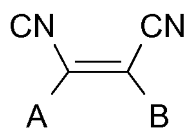

Ils peuvent également former un cycle à 5 ou 6 atomes de carbone, éventuellement fluoré ou perfluoré, notamment selon la formule suivante :

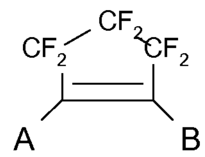

ou former un cycle à 5 atomes de carbone d'anhydride, et notamment selon la formule suivante :

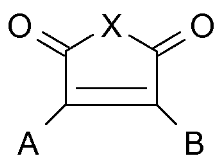

dans laquelle X peut être un atome d'oxygène ou un radical $-NR_3$, avec R3 représentant un radical alkyle et/ou hydroxyalkyle en C2-C16.

Les radicaux A et B peuvent être égaux ou différents et peuvent représenter notamment un cycle à 5 atomes ou un bicycle à 5 et 6 atomes, selon les structures suivantes :

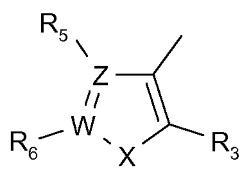

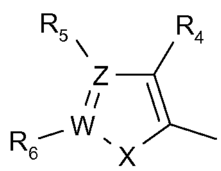

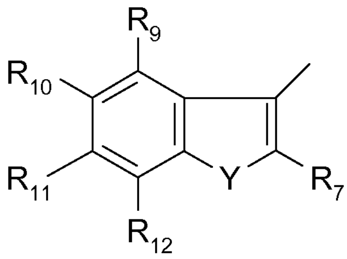

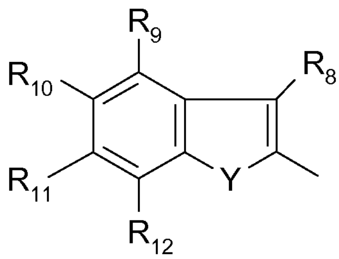

dans lesquelles :

- X et Y peuvent être égaux ou différents, et peuvent représenter un atome d'oxygène, de soufre, d'une forme oxydée du soufre, d'azote ou de sélénium,
- Z et W peuvent être égaux ou différents, et peuvent représenter un atome de carbone ou d'azote,
- les radicaux $R_3$ à $R_{12}$ peuvent être égaux ou différents, et peuvent représenter un hydrogène, un groupe alkyle ou alkoxy, linéaire ou ramifié, en $C_1$-$C_{16}$, un halogène, un groupe fluoré ou perfluoré linéaire ou ramifié en $C_1$-$C_4$, un groupe carboxylique, un groupe alkyle carboxylique en C1-C16, un groupe mono- ou dialkylamino en C1-C16, un groupe nitrile; un groupe phényle, naphtalène ou un hétérocycle (pyridine, quinoléïne, thiophène) peut être substitué sur ces radicaux.

Toutefois, les groupes A et B ne doivent pas être tous les deux égaux à une structure de type indolique telle que ci-dessous :

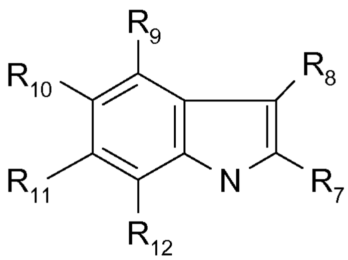

Les groupes A et B peuvent être séparés du cycle par une ou deux doubles liaisons.

Il doit toujours y avoir sur les positions ortho de la jonction, entre la double liaison et les restes A et B, un groupe différent de l'hydrogène, par exemple CH3, CN ou CO2Et, c'est-à-dire que les groupes R3 ou R5, R4, R7 et R8 doivent être différents de la signification hydrogène.

A titre d'exemple, on peut citer le composé suivant qui peut passer de l'incolore au rouge de la manière suivante, après irradiation à 404-436 nm (retour à 546-578 nm) :

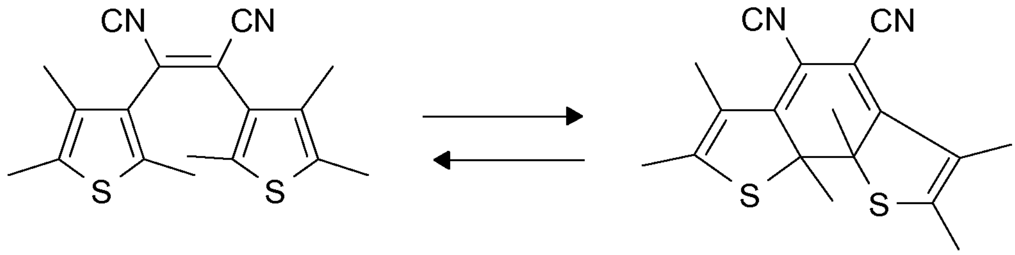

Un exemple de diaryléthène est celui, de couleur bleue à l'état révélé, commercialisé sous la référence commerciale : DAE-MP par la société Yamada Chemical (Japon), de nom chimique et de formule: 1, 2bis(2-methyl-5-phenyl-3-thienyl)-3,3,4,4,5,5-hexafluorocyclopentene

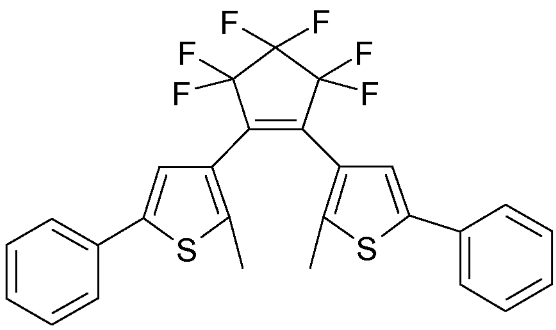

Un autre exemple de diarylethène est celui de couleur jaune à l'état révélé, de formule

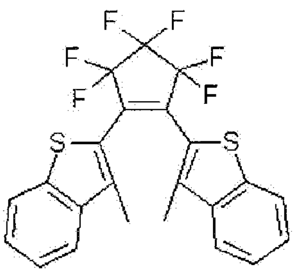

commercialisé sous la référence commerciale : DAE-2BT par la société YAMADA CHEMICALS (Japon) et de nom chimique : 1,2-Bis(3-methylbenzo(b)thiophen-2-yl)perfluorocyclopentene.

Les fulgides peuvent être représentés par la formule suivante :

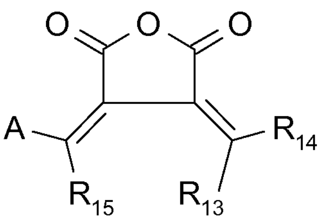

dans laquelle:

- le groupe A a la même signification que ci-dessus.
- les groupes $R_{13}$ à $R_{15}$ peuvent être égaux ou différents, et peuvent représenter un groupe alkyle linéaire ou ramifié en $C_1$-$C_{16}$, ou bien les groupes $R_{13}$ et $R_{14}$ peuvent former un cycle de 3 à 12 atomes de carbone tel qu'un cyclopropane ou un adamantylène.

Autres agents photorévélables thermiquement stables

Ces composés photorévélables sont des composés changeant d'aspect, par exemple passant d'une forme incolore ou peu colorée à une forme colorée, par un mécanisme commandé par une irradiation lumineuse.
Le mécanisme peut être direct, au sens que la lumière fait changer d'aspect, par exemple fait passer de la forme incolore à la forme colorée. C'est par exemple le cas des composés porteur d'une fonction dite photodégradable ou photodétachable (en anglais *photorelease*).
De préférence, on utilise des composés dont la fonction photodégradable ou photodétachable est inerte vis-à-vis des matières kératiniques.
De préférence, on utilise un composé dont la fonction photodégradable ou photodétachable est immobilisée ou portée par un polymère ou autre structure solide ou massique.
Par exemple, on peut utiliser la fonction 3-5diméthoxybenzoine et les produits colorés tels qu'ils sont décrits dans l'article: C P McCoy et al., J. Am. Chem. Soc., 2007, 129, 9572.
Le mécanisme précité peut aussi être indirect, au sens que la lumière fait passer le composé, par exemple initialement incolore, sous une autre forme, puis une tierce action transforme ce composé ainsi incolore modifié sous une forme d'aspect différent, par exemple colorée.
Le mécanisme est indirect également lorsqu'une tierce action agit différement sur le composé non modifié par la lumière et sur le composé modifié, et que par exemple le composé non modifié est transformé et change d'aspect, devient par exemple coloré, tandis que le composé modifié conserve son aspect, par exemple incolore.
Par exemple, on peut utiliser le principe de la diazotypie, qui met en jeu un composé sel de diazonium et un autre composé aromatique, capable de réagir par réaction de couplage. L'irradiation détruit le sel de diazonium (libération d'azote). Le composé diazonium n'ayant pas été irradié réagit ensuite par un simple saut de pH (en présence d'ammoniaque par exemple) avec le coupleur pour donner un composé azoique. Dans ce cas, on choisira des composés sels de diazonium non colorés, comme par exemple un aromatique portant la fonction diazonium mais ne portant pas sur le cycle de fonction amine ou hydroxyle. On peut prendre comme coupleur une amine aromatique simple comme un dérivé d'aniline ou de phénol.
Ce sont alors les parties non irradiées qui se révèlent, selon la réaction :

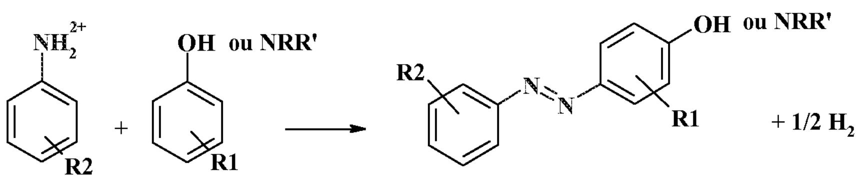

Le ou les agents photorévélables utilisés peuvent devenir thermiquement stables dès leur révélation ou seulement après qu'une action spécifique ait été exercée, par exemple la mise en présence avec des composés chimiques conférant la stabilité thermique recherchée.
La composition photorévélable thermiquement stable peut contenir en masse de 0,001 à 20% au total d'agent (s) photochrome (s), notamment d'agent(s) photorévélable (s) thermiquement stable(s).
La composition photochromique peut contenir en plus tout solvant approprié à l'application en cosmétique, et notamment choisi parmi ceux mentionnés dans le brevet EP 938 887.
La composition peut comporter les ingrédients énumérés aux paragraphes [0029] à [0041] de EP 0 938 887 A1, dont la liste est ici reprise par renvoi.
Composition photorévélable thermiquement stable à sensibilité réduite La composition photorévélable thermiquement stable peut inclure au moins un agent optique réduisant sa sensibilité au rayonnement UV ou proche UV.
La composition photorévélable thermiquement stable peut notamment comporter un ou plusieurs agents optiques en une quantité suffisante pour que son pouvoir filtrant F, tel que défini ci-dessous, soit supérieur ou égal à 2, voire 5, 10, 15 ou 20.

Protocole de mesure du pouvoir filtrant

On met en oeuvre un protocole similaire à celui utilisé pour déterminer le SPF, à la différence que l'on ne tient pas compte de la réponse érythémale de la peau.

On applique la composition dont on cherche à connaître le pouvoir filtrant à raison de 1,2 mg/cm$^2$ sur une plaque sablée de PMMA (sans filtre UV) de 5cm sur 5cm, de 3mm d'épaisseur, de rugosité 4,5 $\pm$ 1 $\mu$m, de la société EUROPLAST. Les plaques sont pré-traitées par un dépôt de 10 $\pm$ 1mg de vaseline 145B. La composition peut être déposée en 14 plots de produit et l'étalement a lieu pendant 20 s au doigt en faisant des zigzags en tournant la plaque d'un quart de tour toutes les 5 s.

Après étalement, il reste 0,6 mg/cm$^2$ de produit. On attend 20 minutes pour le séchage puis on effectue un nouvel étalement.

On utilise un spectroradiomètre (par exemple à sphère d'intégration de la marque Labsphere UV transmittance analyser), lequel mesure la transmittance diffuse de 290nm à 400nm. Chaque valeur de transmission T($\lambda$) est enregistrée. T($\lambda$) est le rapport, pour une irradiation de longueur d'onde $\lambda$ donnée, de l'énergie lumineuse transmise sur l'énergie lumineuse incidente. On fait 5 mesures par plaque (en déplaçant les plaques) et on fait la moyenne de ces 5 mesures. On fait l'opération sur 5 plaques. On réalise les moyennes des 5 moyennes de mesure.

Le pouvoir filtrant F vis-à-vis du rayonnement UV solaire (290 à 400 nm) est donné par le rapport des deux intégrales suivantes:

$$F = \frac{\int_{290\,nm}^{400\,nm} I(\lambda)\,d\lambda}{\int_{290\,nm}^{400\,nm} I(\lambda)T(\lambda)\,d\lambda}$$

où I($\lambda$) est une fonction représentant l'occurrence de chaque longueur d'onde du spectre solaire. I ($\lambda$) est le même que celui servant au calcul de SPF in vitro dans la publication COLIPA GUIDELINES Edition of 2007 A METHOD FOR THE IN VITRO

DETERMINATION OF UVA PROTECTION PROVIDED BY SUNSCREEN

PRODUCTS. Si F=1, alors la composition n'est pas filtrante.

Par « faire écran au rayonnement de longueur d'onde $\lambda$ », il faut comprendre que l'agent optique atténue d'au moins un facteur d'atténuation de 2 le rayonnement de longueur d'onde $\lambda$, la mesure étant effectuée par le biais d'un appareil apte à mesurer le spectre d'absorption en restreignant la lumière d'irradiation à une zone de longueur d'onde $\lambda \pm 10$ nm. Le rapport $F_{\lambda-10,\lambda+10} = \frac{\int_{\lambda-10}^{\lambda+10} I(\lambda)\,d\lambda}{\int_{\lambda+10}^{\lambda+10} I(\lambda)T(\lambda)\,d\lambda}$ avec *I*($\lambda$) et *T*($\lambda$) tels que définis plus haut, donne le facteur d'atténuation à la longueur d'onde $\lambda$.

La composition photorévélable thermiquement stable peut présenter au moins une plage P de longueurs d'onde dans un intervalle $\lambda_1$ à $\lambda_2$ où l'irradiation est moins filtrée, le pouvoir filtrant dans cette plage valant en moyenne $F_{\lambda1,\lambda2}$ avec $F/F_{\lambda1,\lambda2} > 2$, de préférence $F/F_{\lambda1,\lambda2} > 5$. La largeur de la plage P peut être inférieure à 80 nm, et de préférence à 40 nm.

$F_{\lambda1,\lambda2}$ est défini par :

$$\frac{\int_{\lambda 1}^{\lambda 2} I(\lambda)\,d\lambda}{\int_{\lambda 1}^{\lambda 2} I(\lambda)T(\lambda)\,d\lambda}$$

et mesuré comme décrit plus haut, en remplaçant les bornes 290 et 400 par $\lambda_1$ et $\lambda_2$, avec $\lambda_2 > \lambda_1$.

La composition photorévélable thermiquement stable peut comporter, le cas échéant, un colorant évolutif, par exemple un colorant dont la couleur se révèle au cours du temps et si possible lentement, par exemple la DHA ou des

polyphénols, aptes à se colorer progressivement au contact de l'air. La composition photorévélable thermiquement stable comporte par exemple un colorant mettant plus de 1/2h à se révéler à 90 %. L'intérêt d'un tel colorant peut être de développer un pouvoir filtrant une fois le photomaquillage réalisé, afin par exemple de retarder la dégradation des motifs du photomaquillage sous l'effet de l'éclairage ambiant.

Seconde couche jouant un rôle d'activateur d'un agent optique présent dans la première couche

Dans un exemple de mise en oeuvre de l'invention, une première couche est constituée par la composition photorévélable thermiquement stable et contient un agent optique sous une forme partiellement ou totalement désactivée ou à l'état de précurseur. Cet agent sous forme désactivée ou à l'état de précurseur n'a pas encore l'activité suffisante pour protéger le résultat du photomaquillage.

L'application d'une seconde couche permet, après le photomaquillage, d'activer l'agent optique désactivé ou d'amener le précurseur sous sa forme d'agent optique efficace pour former écran au rayonnement de révélation de la composition photorévélable thermiquement stable.

Par exemple, l'agent optique peut, sous une forme précurseur dans l'une des couches, être un colorant de la classe des porphyrines, rendu plus actif par la présence dans l'autre couche d'un sel en solution, par exemple un sel de zinc, de fer ou de magnésium.

Formes galéniques

La composition photorévélable thermiquement stable peut se présenter en fonction des applications et de la nature de ses constituants sous diverses formes galéniques.

La composition photorévélable thermiquement stable selon l'invention contient un milieu cosmétique acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage. Ledit milieu peut comprendre ou se présenter sous la forme de, notamment, une suspension, une dispersion, une solution en milieu solvant ou hydroalcoolique, éventuellement épaissie voire gélifiée ; une émulsion huile-dans-eau, eau-dans-huile, ou multiple ; un gel ou une mousse ; un gel émulsionné ; un spray ; une poudre libre, compacte ou coulée ; une pâte anhydre ; un film, entres autres.

**Zones traitées**

Toutes les parties du corps habituellement maquillées peuvent recevoir un photomaquillage selon l'invention, par exemple les ongles, les cils, les cheveux, la peau, et en particulier celle du visage, par exemple les joues, le front, le contour des yeux ou les lèvres, le cou, le buste ou les jambes.

On peut aussi traiter des parties du corps rarement maquillées comme les oreilles, les mains ou les dents. Ces zones présentent des géométries complexes, ce qui ne facilite pas l'application précise des produits de maquillage conventionnels. Le photomaquillage peut permettre l'obtention de résultats esthétiques, et ce malgré la complexité des géométries.

Le photomaquillage peut viser à camoufler un défaut cutané.

Le photomaquillage peut éventuellement reprendre un motif d'un vêtement ou d'un accessoire porté par l'utilisateur, par exemple un motif présent sur un bijou, un sac à main, des lunettes, des chaussures, un élément de mobilier, un PDA ou un téléphone mobile.

Le cas échéant, la vente d'un tel vêtement ou accessoire peut être accompagnée de la fourniture d'un fichier ou d'un lien internet permettant de réaliser un photomaquillage en accord avec l'accessoire ou le vêtement.

Le fichier ou le lien internet peut donner accès aux données nécessaires pour la réalisation d'une image qui a été conçue ou sélectionnée pour se marier harmonieusement avec le vêtement ou accessoire. Par exemple, elle reprend tout ou partie des motifs ou la complète.

**Description des figures**

- la figure 1 représente de façon schématique et partielle un exemple de système de photomaquillage réalisé conformément à l'invention,
- la figure 2 illustre le maintien de la zone à traiter durant l'irradiation lumineuse,
- la figure 2A illustre la formation d'un motif au sein d'une image pixellisée,
- les figures 3 à 6 sont des vues schématiques et partielles de variantes de réalisation de l'irradiateur,

- les figures 7 à 10 et 10A illustrent différents exemples d'imageurs matriciels adressables selon plusieurs technologies,
- les figures 11 à 13 illustrent des exemples de photomaquillage,
- les figures 14a à 14c et 15a à 15c illustrent des exemples de progression du photomaquillage, et
- la figure 16 représente un exemple de dispositif de conditionnement permettant de révéler la composition avant son application.

**Modes d'application**

La ou chaque composition utile à la mise en oeuvre de l'invention peut être appliquée sous forme pulvérulente, de fluide, de spray ou de film. Le fluide peut avoir différentes rhéologies. Il peut s'agir par exemple d'un bloc de produit s'étalant lors du frottement sur les matières kératiniques ou d'un liquide.

La couche de composition photorévélable thermiquement stable ou photoprotectrice éventuelle, et plutôt la couche de composition photoprotectrice, peut être appliquée sous forme de poudre sèche ou presque sèche.

La ou chaque composition peut éventuellement être sous forme de film préformé.

De préférence, dans le cas d'une application multicouche, la seconde couche est de préférence appliquée sans détériorer la première couche précédemment appliquée. A cet effet, l'application par pulvérisation de la composition destinée à former la seconde couche peut être préférée.

L'application peut encore se faire à l'aide d'une imprimante, à jet d'encre par exemple, grâce à un appareil amené au contact de la zone à traiter, et éventuellement déplacé sur celle-ci. Dans le cas où une ou plusieurs couches sont pulvérisées, toute technique de pulvérisation est utilisable, par exemple gaz propulseur, aérographe ou pulvérisation électrostatique ou piézoélectrique. L'application peut encore se faire par transfert, grâce à une feuille de support portant l'une au moins des compositions, voire les différentes couches à former. Le transfert peut se faire par pression, chaleur et/ou à l'aide d'un solvant, déposé sur la feuille de support et/ou les matières kératiniques devant être traitées selon l'invention.

L'application peut se faire manuellement ou de façon automatique, à l'aide d'un bras manipulateur par exemple.

L'application de chaque couche peut se faire après séchage de la couche éventuelle qui la précède.

L'application peut se faire à l'aide d'applicateurs, éventuellement à usage unique, comportant un organe d'application chargé de la composition à appliquer.

L'application des compositions peut se faire sur un point de vente, dans un salon esthétique, ou à domicile, entre autres lieux.

Chaque composition peut être conditionnée avant l'utilisation dans tout récipient adapté.

L'application des compositions, et notamment de la composition photorévélable thermiquement stable, peut se faire après vérification que la lumière ambiante n'a pas une intensité préjudiciable à la qualité du photomaquillage. La vérification peut s'effectuer grâce à un dispositif avertisseur alertant l'utilisateur si la lumière ambiante contient un rayonnement UV ou proche UV trop fort, par exemple de fluence supérieure ou égale à 0,1 ou 0,5 $mW/cm^2$, le seuil pouvant être réglable le cas échéant. Un tel dispositif peut être autonome ou intégré à un dispositif de conditionnement ou d'application de la composition photorévélable thermiquement stable, voire à un dispositif de conditionnement ou d'application d'une composition photoprotectrice.

L'information délivrée par le dispositif avertisseur peut aussi être utile, le cas échéant, pour sélectionner une composition photorévélable thermiquement stable, photoprotectrice et/ou servant de couche de base parmi plusieurs, en fonction du niveau du rayonnement UV.

Comme évoqué plus haut, la composition photorévélable thermiquement stable peut, selon les cas, être appliquée à l'état complètement révélé ou non révélé.

La composition photorévélable thermiquement stable et celle destinée à former la couche de revêtement ou la couche de base ou la couche photoprotectrice peuvent se présenter sous diverses formes, par exemple crèmes, gels, liquides, sous forme de compositions à étaler à la main, ou par le biais d'un applicateur, par exemple à bille.

L'application peut se faire en déplaçant un bloc de composition au contact des matières kératiniques, comme un bâton de rouge à lèvres par exemple. On peut encore appliquer la composition par pulvérisation en utilisant un bidon aérosol, un flacon pompe ou un dispositif de pulvérisation électrostatique, piézoélectrique ou aérographe.

La composition peut être un produit sec, comme une poudre, dont l'application peut se faire avec une brosse ou un pinceau si besoin.

Lorsque la composition photorévélable est destinée à être appliquée à l'état révélé, elle peut être contenue dans un dispositif de conditionnement et d'application tel qu'illustré à la figure 16, comportant un récipient 1000 contenant la composition, une source lumineuse 1010 émettant par exemple dans l'UV, pour révéler la composition, et un moyen d'application, par exemple un pinceau applicateur 1020.

## Système de photomaquillage

Le photomaquillage peut être réalisé à l'aide d'un système de photomaquillage 1 comportant, comme illustré schématiquement à la figure 1, un irradiateur 3 comportant au moins une source lumineuse 2. L'imageur peut, selon les cas, servir à révéler la composition photorévélable thermiquement stable, en faisant par exemple passer le ou les agents photorévélables qu'elle contient d'un état non révélé à l'état révélé et/ou servir à effacer le ou les agents photorévélables que contient la composition photorévélable thermiquement stable, en les faisant passer d'un état révélé à un état non révélé. Lorsque la composition est initialement à l'état non révélé, l'irradiateur peut par exemple émettre dans l'UV ou le proche UV lorsque la composition photorévélable thermiquement stable est révélable par un rayonnement UV ou dans le proche UV.

L'image peut être définie par un masque ou un négatif disposé sur le trajet de la lumière vers la zone à traiter, éventuellement en contact avec la zone à traiter.

L'irradiateur comporte de préférence un ou plusieurs imageurs 4 permettant de former au moins une image à distance sur la zone à traiter Z.

Le ou les imageurs peuvent, dans une version simplifiée, utiliser un masque ou un négatif et une optique de projection sur la zone à traiter. Le négatif peut être un négatif UV, permettant la formation d'une image en lumière UV ou proche UV.

La source 2 peut comporter tout type d'élément lumineux, par exemple lampe à incandescence, lampe halogène, lampe à décharge et/ou élément électroluminescent, notamment une ou plusieurs LEDs, OLED ou autres technologies électroluminescentes.

L'irradiateur 3 peut comporter, comme cela sera détaillé plus loin, plusieurs sources afin d'émettre d'une part dans l'UV ou le proche UV et d'autre part dans le visible, notamment hors du proche UV.

Avantageusement, l'irradiateur comportant la ou les sources de lumière et le ou les imageurs peut émettre sélectivement dans l'UV ou le proche UV et le visible.

Le passage d'une projection d'image en lumière visible à une projection d'image en lumière UV peut se faire par un changement de source, grâce par exemple à un miroir mobile, à l'utilisation d'une surface semi-transparente, à l'ajout ou au retrait d'un filtre et/ou à l'utilisation d'un doubleur ou tripleur de fréquence.

L'emploi de lumière visible peut permettre, comme cela sera détaillé dans la suite, de voir l'image projetée sur la zone à traiter avant de réaliser la révélation et/ou de faire régresser le photomaquillage d'une zone déjà révélée au moins partiellement, quand le ou les agents photorévélables le permettent.

Le système de photomaquillage peut comporter, comme illustré à la figure 1, un calculateur 10, qui peut être associé à une interface utilisateur 11, comportant par exemple un clavier, une souris, un écran tactile, un moteur de reconnaissance vocale, une tablette graphique, un joystick et/ou un pad tactile, cette liste n'étant pas limitative.

Le calculateur 10 peut comporter un microordinateur et, de façon plus générale, tout moyen de calcul, analogique et/ou numérique, réalisé à l'aide par exemple de microcontrôleurs, microprocesseurs et/ou réseaux logiques programmables.

Le calculateur 10 peut être réalisé sous la forme d'un ou plusieurs appareils et dans le cas de l'utilisation d'un imageur électronique, ce dernier peut le cas échéant réaliser tous les calculs ou une partie de ceux-ci. Le calculateur 10 peut également être associé à un moyen de visualisation 12 qui est par exemple un écran en couleur, par exemple de type LCD, plasma, OLED ou cathodique, éventuellement tactile. Ce moyen de visualisation 12 peut être utilisé pour, comme détaillé dans la suite, afficher la zone traitée en cours de traitement, permettre de commander le traitement et/ou afficher une simulation.

Le calculateur 10 peut encore être associé à un moyen de stockage de données 13, par exemple à disque dur, bande magnétique, disque optique et/ou mémoire flash, le moyen de stockage de données pouvant être intégré au calculateur 10, à l'irradiateur 3 et/ou au moins partiellement déporté au sein d'un système de stockage de données externe.

Le calculateur 10 peut être associé à une interface réseau 14 qui peut permettre par exemple de télécharger des données utiles au photomaquillage ou de transmettre à des tiers ou à un serveur des données concernant un photomaquillage en cours ou réalisé.

Le calculateur 10 peut avantageusement commander, le cas échéant, la ou les sources produisant la lumière utilisée pour former l'image, afin que l'image soit formée avec un illuminant prédéfini.

L'imageur est avantageusement un imageur électronique matriciel adressable, tel que décrit plus loin, auquel le calculateur peut adresser des données afin de provoquer la projection sur la zone Z à traiter d'une image prédéfinie.

Le système de photomaquillage peut être doté d'au moins un système optique et/ou électronique permettant de focaliser l'image, de façon manuelle ou automatique et avantageusement de moyens permettant d'éviter les bougers.

Pour immobiliser la zone en cours de traitement relativement à l'irradiateur, le système de photomaquillage peut comporter des moyens d'immobilisation de la personne, permettant d'éviter les bougers et les résultats flous.

Lorsque l'image est formée sur un visage, le système de photomaquillage peut être configuré pour détecter

que le visage est au repos, et commander l'imageur en fonction de cette détection.

Le système de photomaquillage 1 peut comporter une partie plane ou pouvant épouser la forme d'une partie du corps et l'on peut plaquer cette partie sur la zone à traiter.

Le système de photomaquillage peut, en variante ou additionnellement, comporter un système de rectification des mouvements, du même type par exemple que ceux utilisés pour stabiliser les images dans les appareils photographiques ou les caméras.

Lorsque le visage est traité, le système de photomaquillage peut comporter un moyen 8 d'immobilisation de la personne à traiter, sous forme d'une mentonnière, comme illustré à la figure 2.

En variante, l'irradiateur 3 est portatif et peut se fixer sur une monture portée par la personne à traiter, afin d'illuminer une zone du visage par exemple.

Le système de photomaquillage 1 peut comporter un dispositif d'acquisition optique 16 qui peut, dans un exemple de mise en oeuvre de l'invention, transmettre des données au calculateur 10 afin que ce dernier puisse proposer un photomaquillage et/ou contrôler la réalisation de celui-ci, comme cela sera détaillé plus loin. Le dispositif d'acquisition optique 16 peut avantageusement avoir un axe de visée sensiblement parallèle à la direction de projection de l'image. Le dispositif d'acquisition optique peut être monopixel ou multipixel, et recevoir directement la lumière émise par l'imageur ou recevoir la lumière réfléchie par la zone Z à traiter.

Le dispositif d'acquisition optique éventuel, l'irradiateur, le calculateur éventuel et l'écran de visualisation éventuel peuvent être réalisés sous forme d'éléments séparés ou intégrés dans un même boitier. L'irradiateur et le dispositif d'acquisition optique peuvent avantageusement être intégrés dans un même boitier ou être rendus solidaires en déplacement autrement.

L'écran de visualisation peut être fixé au dos du boitier de l'irradiateur ou intégré dans le boitier. Le cas échéant, le dispositif d'acquisition optique comporte un moyen d'éclairage interne pour une acquisition de près.

Le boitier de l'irradiateur peut encore être mobile et être appliqué sur la peau par exemple, ou être tenu à la main. Dans un exemple de mise en oeuvre de l'invention, le boitier de l'irradiateur peut être posé, par exemple sur une table. Dans ce cas, on peut approcher le visage pour le poser sur le boitier, en se penchant par exemple.

Le système de photomaquillage peut être doté de moyens pour détecter l'ouverture ou la fermeture des yeux et/ou de la bouche, afin d'arrêter ou ne pas lancer l'irradiation en cas d'ouverture des yeux et/ou de la bouche. Le dispositif d'acquisition optique 16 peut fournir une image qui est analysée à cet effet par le calculateur 10.

Lorsque l'image est formée sur un visage, le système de photomaquillage peut être configuré pour identifier le visage et l'imageur peut être commandé en fonction au moins de cette identification.

Le système de photomaquillage est avantageusement conçu pour permettre à l'utilisateur d'évaluer, de façon visuelle ou non, l'évolution du photomaquillage.

Le système de photomaquillage peut comporter à cet effet une fenêtre permettant une visualisation directe de la zone traitée en cours d'irradiation, cette visualisation s'effectuant le cas échéant au travers d'un filtre UV. Afin de laisser la place à une visualisation directe, l'émission de la lumière peut se faire depuis un emplacement déporté et l'on peut employer pour conduire la lumière et focaliser les rayons lumineux sur la zone à traiter, des fibres optiques ou au moins un miroir ou un prisme, par exemple.

On a représenté partiellement et schématiquement à la figure 3 un système de photomaquillage qui comporte deux imageurs 4a et 4b, émettant respectivement des lumières UV et visible, vers la zone Z à traiter. Une fenêtre 103 est ménagée entre ces imageurs 4a et 4b, pour permettre une observation de la zone Z en cours de traitement.

On peut encore déporter la zone de visualisation grâce à un miroir 404 ou tout autre système optique, par exemple à fibre optique ou à prisme, comme illustré à la figure 4.

En présence d'un dispositif d'acquisition optique tel qu'une caméra vidéo ou un appareil photographique numérique, la visualisation de la zone traitée Z peut se faire sur un écran qui peut être placé sur l'irradiateur ou être déporté.

Sur la figure 5, on a illustré la possibilité de réaliser l'irradiateur 3 en déportant à l'aide de miroirs 18 le faisceau lumineux dirigé vers la zone Z à traiter, ce qui peut permettre une observation par l'utilisateur de la zone Z traitée au travers d'une fenêtre 20 de l'irradiateur.

Sur la figure 6, on a illustré la possibilité de réaliser l'irradiateur 3 avec deux sources lumineuses 2a et 2b émettant respectivement dans l'UV et le visible. L'irradiateur représenté à la figure 6 comporte un filtre coloré 302, par exemple vert, placé devant la source 2b, une optique de collimation réglable 303 et un miroir mobile 304. L'irradiateur 3 permet dans cet exemple de placer un négatif 308 sur le chemin optique. L'optique de collimation réglable 303 permet de faire apparaître l'image du négatif à une certaine distance de la sortie optique de l'irradiateur 3, par exemple une vingtaine de centimètres.

L'irradiateur 3 est doté de deux interrupteurs 306 et 307. Le premier met en action les sources 2a et 2b. Le miroir mobile 304 est disposé de façon que seule l'irradiation en lumière visible soit dirigée vers la sortie optique pour une position donnée du second interrupteur. L'actionnement de ce dernier déplace le miroir mobile, par exemple en actionnant un micromoteur ou un électroaimant, et l'irradiation UV est alors dirigée vers le négatif 308.

Le système de photomaquillage comporte avantageusement, comme mentionné plus haut, un imageur électronique matriciel adressable.

Imageur électronique matriciel adressable

Un imageur matriciel adressable permet de projeter une image pixellisée dont la résolution est par exemple supérieure à 10 par 10 pixels et préférentiellement supérieur à 10 par 100 pixels.

Lorsque l'imageur est un imageur électronique matriciel adressable, l'image formée sur la zone à traiter est formée de pixels qui sont allumés ou éteints, éventuellement chacun selon un niveau de gris prédéfini. A titre d'exemple, on a représenté à la figure 2A un détail de la figure 2 où le photomaquillage P qui est réalisé consiste en un contour des lèvres. On a représenté sur la figure 2A l'emplacement des différents pixels de l'image projetée, seuls les pixels correspondant au contour à réaliser ayant été allumés. La révélation de la composition photorévélable thermiquement stable se fait en correspondance avec l'état des pixels.

La lumière sortant de l'imageur matriciel adressable peut être monochromatique ou multichromatique, et de préférence l'imageur matriciel adressable est capable d'émettre sélectivement d'une part dans l'UV ou le proche UV et d'autre part dans le visible hors du proche UV, la lumière émise dans le visible pouvant être de la lumière blanche ou une lumière colorée, éventuellement monochromatique.

Le calculateur 10 peut déterminer l'image numérique sur la base de laquelle l'imageur électronique est commandé, en particulier le niveau de gris de chaque pixel, ainsi éventuellement que la longueur d'onde dominante de la lumière au niveau de chaque pixel.

L'imageur matriciel adressable peut être réalisé sur la base de plusieurs technologies.

On peut employer la technologie dite DLP (*Digital Light Proccessing*) inventée par la société TEXAS INSTRUMENTS, qui utilise une puce DMD (*Digital Micromiror Device*) composée de milliers de micromiroirs qui peuvent être commandés individuellement en orientation sous l'effet d'une impulsion électrique et peuvent réfléchir ou non suivant leur orientation un faisceau lumineux incident afin de le renvoyer ou non vers la sortie optique de l'imageur. L'image à projeter est formée sur la matrice de miroirs. Les niveaux de gris de chaque pixel (par exemple au nombre de 256 niveaux) peuvent être commandés en jouant sur le rapport cyclique.

La figure 7 représente un exemple de réalisation d'un imageur électronique 4 réalisé selon cette technologie, utilisant une puce DMD référencée 111. Cette dernière peut être fixée sur une platine 112 qui peut comporter par ailleurs un processeur 113 pour commander la puce, ainsi qu'éventuellement une mémoire 114. Dans l'exemple illustré, la puce est représentée sur la même platine que le processeur 113 et la mémoire 114, mais ces derniers peuvent être disposés autrement.

L'imageur 4 représenté à la figure 7 reçoit de la lumière d'une source 2 qui peut être une source pouvant émettre à la fois dans l'UV et/ou dans le visible ou une source pouvant émettre sélectivement dans le visible ou dans l'UV.

La source 2 peut être une lampe halogène à émission dans les spectres UV et visible, une lampe à décharge ou une ou plusieurs LEDs capables d'émettre par exemple dans l'UV et en lumière blanche ou dans une couleur donnée.

L'imageur 4 peut comporter, comme illustré, des optiques 118, 119 et 120 respectivement pour condenser la lumière, la focaliser sur la puce DMD et assurer la mise au point sur la zone à traiter.

Lorsque la source 2 présente un spectre d'émission à la fois dans l'UV et dans le visible, l'imageur 4 peut comporter, comme illustré, une roue à filtres 30, qui intersecte le faisceau lumineux par exemple entre l'optique de condensation 118 et celle de focalisation 119. La puce reçoit selon la position de la roue à filtres 130 une lumière UV ou une lumière visible, qui est ensuite dirigée vers la sortie optique. On peut ainsi former sur la zone à traiter une image sélectivement en lumière visible et/ou dans l'UV.

L'irradiateur selon la variante de la figure 8, utilise plusieurs puces DMD fixées sur un prisme qui divise la lumière incidente provenant de la source 2 en au moins deux faisceaux ayant des longueurs d'onde dominantes différentes, par exemple respectivement dans l'UV ou le proche UV et dans le visible.

Les faisceaux lumineux réfléchis par les puces DMD sont projetés vers la zone à traiter.

En pilotant la puce DMD associée au faisceau UV ou proche UV et celle associée au faisceau de lumière visible, on peut projeter sur la zone à traiter soit une lumière UV, soit une lumière visible, soit éventuellement les deux à la fois, ce qui peut être utile lorsque la révélation est relativement lente, afin de pouvoir contrôler visuellement le bon positionnement de la lumière servant à la révélation.

L'irradiateur peut encore utiliser la technologie dite LCD.

Dans l'exemple de la figure 9, la source 2 est dirigée sur des miroirs dichroïques 125 qui génèrent au moins deux faisceaux lumineux de différentes longueurs d'onde dominantes, l'un de ces faisceaux ayant par exemple une longueur d'onde dominante dans l'UV ou le proche UV et l'autre dans le visible.

Les faisceaux sont dirigés par des miroirs 125 et 126 vers des écrans 127 à matrice LCD sur laquelle est formée l'image à projeter, qui produisent des images monochromes vers un système de prismes 128, lequel permet de

renvoyer l'image, par l'intermédiaire de l'optique de projection 120, vers la surface à traiter. Selon le degré d'opacité des écrans 127, la lumière émise est une lumière dans le visible ou dans l'UV.

L'irradiateur 3 illustré à la figure 10 comporte un écran 132 à matrice LCD, et une source 2 qui éclaire l'écran 132. L'image formée sur ce dernier est projetée, grâce à l'optique de projection 120, sur la zone à traiter. La source 2 est par exemple capable d'émettre sélectivement dans l'UV ou dans le visible.

L'écran 132 peut aussi, dans une variante, remplacer le négatif 308 de l'exemple de la figure 6.

Le système de projection peut encore être fondé sur la technologie dite LCOS à cristaux liquides sur silicium. La technologie LCD est dite transmissive puisque la lumière traverse un écran LCD alors que la technologie DLP est dite réflective, car la lumière est reflétée par les micromiroirs de la puce DMD. Dans la technologie LCOS, les miroirs des puces DMD sont remplacés par une surface réfléchissante recouverte d'une couche de cristaux liquides, qui peuvent être commutés entre un état passant et un état bloquant. En modulant la fréquence d'ouverture et de fermeture des cristaux liquides, on peut faire varier le niveau de gris d'un pixel.

On peut utiliser les agencements illustrés aux figures 7 et 8 par exemple, en remplaçant les puces DMD par des puces LCOS.

La figure 10A représente un irradiateur à puce LCOS. Un système de lentilles 901 peut être disposé entre la source 2, par exemple une lampe à UV, et un miroir semi-transparent 903. Ce dernier réfléchit la lumière provenant de la source vers la puce 900. Cette dernière réfléchit à nouveau la lumière vers un système de focalisation 120, qui projette l'image pixellisée sur la zone à traiter.

D'une manière générale, l'image délivrée par l'imageur matriciel adressable comporte une matrice de pixels dont les niveaux de gris sont individuellement adressables, chaque niveau de gris étant par exemple codé sur au moins 4 bits, mieux 8 bits. La lumière associée à chaque pixel peut également faire l'objet d'un codage, le cas échéant.

L'image à projeter peut être fournie à l'imageur électronique sous forme de signal vidéo VGA, SVGA, composite, HDMI, SVIDEO, $YC_BC_R$, optique, entre autres standards, ou sous forme de fichier informatique image ou vidéo, par exemple .jpeg, .pdf, .ppt, ... Sur ces images, lorsqu'elles ne sont pas monochromes, une couleur prédéfinie sur l'image dans le fichier peut commander le niveau d'UV ou de proche UV, par exemple.

L'imageur électronique est avantageusement réalisé de façon à pouvoir changer la nature de la lumière émise sans changer l'image ; par exemple, les pixels de l'image conservent leurs niveaux de gris et seul le spectre d'émission de la source utilisée en amont change. Cela peut permettre de visualiser une image sur la zone à traiter et ensuite de la révéler, simplement en modifiant le spectre d'émission de la source.

L'imageur peut être utilisé pour projeter une lumière visible afin d'effacer sélectivement un ou plusieurs agents photorévélables et réaliser un photomaquillage à partir d'une couche de composition photorévélable thermiquement stable à l'état révélé. Dans ce cas, l'imageur est par exemple un vidéoprojecteur conventionnel.

<u>Choix de l'image projetée</u>

Notamment lors de l'utilisation d'un imageur électronique adressable, le système de photomaquillage est préférentiellement doté d'un moyen permettant de choisir l'image projetée. Ceci peut se faire grâce à un choix dans une bibliothèque d'images, avec éventuellement un défilement de plusieurs images de cette bibliothèque et sélection par l'utilisateur d'une image affichée. Les images peuvent être stockées sous forme digitalisée ou photographique, par exemple dans le moyen de stockage de données. La bibliothèque d'images peut être incluse dans le système de photomaquillage ou être téléchargeable.

Dans un exemple de mise en oeuvre l'invention, on utilise une image faite sur mesure à partir de la personne destinée à recevoir le photomaquillage, ou d'un modèle tel qu'une célébrité ou une personne d'esthétique donnée, les images pouvant être issues de personnes non maquillées ou maquillées. On peut aussi utiliser des images issues de dessins, tableaux, croquis ou caricatures pour générer l'image projetée.

Le calculateur peut avoir en mémoire ou peut charger au moins un modèle pictural, sous forme par exemple de trait ou de touche de couleur, voire un simple point ou une série de traits, touches ou points.

L'image formée peut être déterminée automatiquement en fonction de l'image acquise. Cela peut permettre d'adapter l'image projetée à la morphologie et/ou à la couleur du visage.

Le calculateur peut, en partant de la position du visage capturée, positionner correctement l'image destinée à réaliser le photomaquillage.

Le système de photomaquillage peut ainsi être utilisé dans un procédé comportant les étapes consistant à :

- acquérir au moins une image de la zone à maquiller du sujet,
- commander l'imageur en fonction de l'image ainsi acquise.

L'acquisition de l'image s'effectue par exemple à l'aide du dispositif d'acquisition optique 16, lequel peut être adapté à capturer l'ensemble ou une partie du visage, ou toute autre zone du corps traitée.

Par exemple, pour réaliser un photomaquillage sur les paupières supérieures, on peut mettre en oeuvre les étapes suivantes :

- capturer l'image du visage, en déduire la zone des paupières,
- la composition photorévélable thermiquement stable ayant été appliquée sur la zone des paupières, irradier le modèle pictural à réaliser à l'endroit même de la zone des paupières ; ainsi, le photomaquillage résultant est formé au bon endroit. L'irradiation à l'emplacement de la zone à traiter peut se faire en allumant seulement les pixels correspondants, dans le cas où l'image est susceptible de couvrir en cas d'allumage de tous les pixels une zone beaucoup plus étendue. Afin de bénéficier de la meilleure résolution, la zone traitée peut concerner par exemple au moins les 2/3 du nombre total de pixels de l'image.

Le calculateur peut aussi modifier la forme du modèle pictural pour l'adapter à la forme du visage. Ainsi, par exemple, si l'on souhaite réaliser un maquillage des lèvres, on peut mettre en oeuvre les étapes suivantes :

- capturer l'image du visage, en déduire la zone des lèvres,
- comparer la forme des lèvres au modèle pictural à reproduire,
- modifier le modèle pictural pour qu'il s'inscrive dans la forme des lèvres,
- une composition photorévélable thermiquement stable ayant été appliquée sur les lèvres, irradier ces dernières pour réaliser le modèle pictural ainsi modifié. Ce procédé peut s'appliquer également à d'autres régions du corps.

Ainsi, le système de photomaquillage peut être doté des quatre fonctions suivantes :

- capturer l'image du visage ou de toute autre région du corps à traiter,
- caler le positionnement du modèle pictural à réaliser sur la partie du visage ou du corps devant le recevoir, et ce en analysant l'image du visage ou de toute autre partie à traiter,
- optionnellement, modifier la forme du modèle pictural pour l'adapter à la forme du visage,
- commander la projection de l'image destinée à réaliser le photomaquillage.

Le système de photomaquillage peut comporter des moyens d'acquisition de la forme 3D du visage. Le système de photomaquillage peut comporter un dispositif d'acquisition optique adapté à détecter le relief, par projection de franges par exemple, et/ou adapté à détecter la brillance.

Dans un exemple de mise en oeuvre de l'invention, le modèle pictural utilisé est déterminé automatiquement. Ce choix peut se faire de façon aléatoire ou en suivant une logique programmée, destinée à optimiser par des règles l'esthétique du visage, pour respecter par exemple une logique d'harmonie des couleurs ou une logique d'harmonie naturelle du visage. Ainsi, par exemple, pour un visage de carnation rousse, il est possible de réaliser des taches de rousseur.

Le choix peut également se faire suivant une logique de rétablissement de la symétrie pour les visages présentant des asymétries et/ou selon une logique d'ombres et de lumières, par laquelle on peut rendre plus rond un visage trop anguleux ou l'inverse, ou rectifier des proportions peu esthétiques ou naturelles.

Dans un exemple de mise en oeuvre de l'invention, le système de photomaquillage propose plusieurs modèles picturaux, laissant à l'utilisateur le loisir d'en sélectionner un. L'expression de ces propositions peut se faire de façon graphique, par exemple par affichage sur un écran. On peut superposer à une image du sujet destiné à recevoir le photomaquillage le modèle pictural proposé, ou ce dernier peut être affiché sur un écran décrivant le visage par un schéma. Toute interface permettant à l'utilisateur de sélectionner un modèle pictural peut être utilisée. Par exemple, la description d'un modèle pictural proposé à l'utilisateur peut se faire de façon verbale, par la description des actions que le système de photomaquillage propose de réaliser.

Le système de photomaquillage peut être configuré pour détecter de façon automatique un défaut cutané sur la zone à traiter et l'imageur peut être commandé en fonction de la nature du défaut détecté.

Le système de photomaquillage peut être doté de fonctions de reconnaissance particulières, destinées par exemple à reconnaître les défauts, par exemple :

- taches, points noirs, boutons, taches de vin, rougeurs,
- rides, crevasses, vergetures, veines,
- reliefs en creux ou en bosse, tels que des cicatrices,
- asymétries,
- desquamation,
- peau mate ou peau brillante,
- poils.

Les défauts peuvent être détectés par analyse d'image et/ou du relief. L'analyse d'image peut être une analyse d'image 3D. L'analyse d'image peut comporter une analyse de la couleur et/ou de la brillance.

Le système de photomaquillage peut aussi être doté de fonctions permettant de calculer ou choisir un modèle pictural destiné à limiter la visibilité de ces défauts. Parmi ces modèles picturaux, on peut citer ceux destinés à flouter certaines parties détectées comme présentant des défauts, et ceux destinés à redessiner certaines parties, dans le cas notamment de cicatrices ou d'asymétries.

Dans un autre exemple de mise en oeuvre de l'invention, l'utilisateur ou une tierce personne peut définir le modèle pictural à réaliser. Ainsi, l'utilisateur ou la tierce personne peut envoyer des commandes qui seront interprétées par le système de photomaquillage. Ces commandes peuvent être graphiques et le système de photomaquillage peut comporter une interface homme machine du type écran tactile. L'utilisateur envoie les ordres de maquillage en désignant sur l'image du visage ou sur un schéma de visage les zones sur lesquelles il veut réaliser un trait de maquillage. Le système de photomaquillage peut être configuré pour interpréter les instructions de l'utilisateur, les adapter à la topographie du visage puis réaliser le photomaquillage.

Les commandes peuvent être des descriptions, par exemple "remplir la zone des lèvres en rouge" ou être intuitives, par exemple "maquillage des paupières". Le système de photomaquillage interprètera alors, de façon conventionnelle ou programmée spécifiquement, qu'il doit adopter un modèle pictural par défaut.

Les commandes peuvent être programmées et les programmes peuvent être personnalisés.

Celui qui choisit parmi les modèles picturaux proposés ou qui détermine les modèles picturaux à réaliser peut être la personne qui se maquille, ou une autre personne, comme un maquilleur professionnel. Le choix ou l'élaboration des modèles picturaux peut se faire dans le même lieu que celui où se fait le photomaquillage, ou à distance. Dans ce dernier cas, le système de photomaquillage peut être doté d'un moyen de communication permettant de communiquer l'image de la zone à traiter, par exemple l'interface réseau 14 précitée.

Le système de photomaquillage peut éventuellement être doté d'un moyen permettant de capturer des maquillages à partir de magazines ou autres supports et d'en faire des modèles picturaux qu'il pourra par la suite reproduire sur la zone à traiter, par exemple un scanner ou un lecteur de puce RFID, la puce contenant la description du maquillage ou un lien internet permettant de la télécharger. Cette puce peut être contenue dans un conditionnement contenant la ou les compositions à utiliser pour réaliser le photomaquillage ou être contenue dans un article vestimentaire ou autre accessoire présentant un motif particulier, qui pourra être reproduit par photomaquillage.

Le système de photomaquillage peut être configuré pour faire défiler toutes sortes de modèles picturaux, sous forme de simulations, afin de permettre à une personne de choisir parmi celles-ci le modèle à réaliser.

Le système de photomaquillage peut offrir la possibilité d'essayer rapidement toutes sortes de modèles, directement sur le visage. Ainsi, la personne peut se rendre compte, en version réelle, de l'adéquation de ce ou ces modèles sur elle. Ces modèles peuvent être des images projetées en lumière visible sur le visage, qui ne révèlent pas la composition photorévélable thermiquement stable, ou des photomaquillages réalisés au moyen de la composition photorévélable thermiquement stable et effaçables, par exemple par irradiation en lumière visible.

Le système de photomaquillage peut avantageusement avoir en mémoire dans les moyens de stockage 13 plusieurs modèles préenregistrés et mémoriser les modèles picturaux qu'il a pu réaliser. De cette façon, l'utilisateur peut utiliser ou échanger les modèles picturaux enregistrés.

Dans un exemple de mise en oeuvre de l'invention, une fois un modèle pictural retenu, l'adaptation du modèle pictural à la topographie du visage de la personne et la réalisation du photomaquillage par projection de l'image sont effectuées automatiquement. Le laps de temps séparant la capture du visage et la réalisation de l'image peut être rendu relativement court, par exemple moins d'une seconde.

Dans un autre exemple de mise en oeuvre de l'invention, la personne recevant le photomaquillage ou une tierce personne peut intervenir pendant le déroulé des opérations. Dans ce cas, la réalisation du maquillage peut être plus lente que précédemment. Le système de photomaquillage peut être configuré de manière à permettre à la personne ou la tierce personne de visualiser la progression du photomaquillage, par exemple sur l'écran 12, afin de ralentir ou d'arrêter son déroulement.

Le système de photomaquillage peut éventuellement régulièrement recapturer le visage de façon à recommencer les opérations de calage et d'adaptation du modèle pictural au visage, éliminant ainsi les éventuels problèmes que pourraient occasionner les mouvements de la personne durant l'irradiation destinée à révéler la composition photorévélable thermiquement stable.

On peut réaliser plusieurs photomaquillages partiels successifs. Ainsi, on peut déterminer, au fur et à mesure du photomaquillage chaque modèle pictural, estimer à l'oeil son rendu, puis choisir le modèle pictural suivant et ainsi de suite, construisant ainsi progressivement le photomaquillage.

Comme mentionné plus haut, le système de photomaquillage peut être configuré pour évaluer, grâce à un ou plusieurs programmes spécialisés, les modèles picturaux les plus adaptés à un visage ou une partie d'un visage. Ainsi, le photomaquillage peut se faire en réalisant un premier modèle pictural, puis en évaluant une seconde fois le visage pour en déduire le nouveau modèle pictural à réaliser, et ainsi de suite.

Il est possible de traiter une partie du visage de façon semi-automatique et une autre de façon automatique. Il est aussi possible de traiter une partie du visage de façon automatique jusqu'à un certain point, puis de poursuivre de façon semi-automatique le photomaquillage, ou inversement.

Le système de photomaquillage peut être configuré pour prendre une image, par exemple à l'aide du dispositif d'acquisition optique précité, en extraire éventuellement une partie correspondant à la zone à traiter, et permettre de rectifier le cas échéant cette image pour en améliorer le rendu une fois projetée.

Le système de photomaquillage utilisé est préférentiellement configuré pour permettre à l'utilisateur, à partir d'une image projetée sur le visage ou sur toute autre zone à traiter, d'en rectifier la forme, par exemple par agrandissement, rétrécissement, dans une ou deux dimensions. Les modifications peuvent aussi être plus complexes. Ainsi, on peut par exemple rectifier une partie de l'image, étirer une zone particulière, changer la taille des traits... En cela, on peut utiliser les outils habituellement présents dans les logiciels de réalisation et de retouche d'image, tels que Photoshop® par exemple. La retouche de l'image peut s'effectuer, le cas échéant, grâce à un renvoi d'information par le dispositif d'acquisition optique, le calculateur pouvant connaître le rendu de l'image projetée et la modifier automatiquement jusqu'à atteindre le résultat souhaité, lors de l'exécution d'une boucle logicielle par le système de photomaquillage.

Réalisation progressive du photomaquillage

Le photomaquillage peut mettre en oeuvre les étapes consistant à :

- appliquer une composition photorévélable thermiquement stable sur une zone à traiter,
- irradier la zone avec une lumière choisie pour révéler progressivement la composition photorévélable thermiquement stable, respectivement pour effacer progressivement la composition photorévélable thermiquement stable,
- interrompre et/ou modifier des caractéristiques de l'irradiation quand l'aspect souhaité est atteint, cet aspect correspondant par exemple à une révélation partielle de la composition photorévélable thermiquement stable, respectivement un effacement partiel de la composition photorévélable thermiquement stable.

On peut ainsi obtenir plus facilement des résultats de maquillage d'intensité dosée. Pendant l'illumination progressive, l'utilisateur et/ou le système de photomaquillage peut surveiller l'avancement du photomaquillage et peut arrêter son évolution au moment où le résultat désiré est atteint.

On peut même procéder, si la composition photorévélable thermiquement stable le permet, à des retouches pour encore affiner le photomaquillage, soit au moment du photomaquillage, soit plus tard.

L'irradiation peut être interrompue puis reprise au moins une fois.

La longueur d'onde dominante et/ou l'intensité de l'irradiation peuvent être modifiées avant que l'aspect souhaité ne soit atteint. Par exemple, en modifiant l'intensité de l'irradiation on peut agir sur la vitesse de révélation ou d'effacement de la composition photorévélable thermiquement stable. En agissant sur la longueur d'onde dominante, on peut agir sur l'énergie de l'irradiation et/ou sur l'effet exercé sur le ou les agents photorévélables.

L'ensemble de l'image peut être traité de façon progressive, mais il est aussi possible de traiter l'image portion par portion de façon progressive, par exemple de façon automatique, programmée ou programmable.

Le photomaquillage peut être utilisé pour créer successivement plusieurs motifs. Au moins un motif ayant atteint l'aspect recherché peut cesser d'être irradié alors qu'au moins un autre motif l'est encore. Les motifs sont par exemple des taches de rousseur, que l'on peut créer successivement.

Ainsi, un programme spécifique peut être exécuté pour réaliser des taches de rousseur, comme représenté à la figure 13. Le programme peut faire apparaître les taches, par le biais d'une irradiation progressive adaptée, soit depuis le centre de la zone vers l'extérieur de la zone (figures 13A à 13C), soit depuis une distribution clairsemée à une distribution dense (figures 14A à 14C), soit depuis une distribution de petites taches à une distribution de grosses taches, soit de façon aléatoire (non illustré).

L'irradiation peut être suffisamment faible pour ne pas amener à une révélation importante dans l'instant suivant son déclenchement.

Afin de permettre une révélation suffisamment lente, l'énergie E de l'irradiation pendant une seconde peut être inférieure ou égale à 0,5 Eo, mieux 0,2 Eo, où Eo est l'énergie nécessaire pendant une seconde pour révéler 80 % de la composition photorévélable thermiquement stable. On peut avoir $E \leq 0{,}2\ E_0$. On considère que l'on a révélé 80 % de la composition photorévélable thermiquement stable quand l'écart de couleur $\Delta E$ avec l'état non révélé correspond à 80 % de l'écart coloriel maximal atteignable.

De même, lorsqu'il s'agit d'effacer, l'énergie E' de l'irradiation pendant une seconde peut être inférieure ou égale à 0,5 $E'_0$, où $E'_0$ est l'énergie nécessaire pendant une seconde pour effacer à 80 % la composition photorévélable thermiquement stable. On peut avoir $E' \leq 0{,}2\ E'_0$.

Le système de photomaquillage peut être configuré pour analyser la couleur de la zone à traiter, puis le résultat de cette analyse peut servir à commander automatiquement l'irradiation. Par exemple, la couleur peut être analysée

après l'application de la composition photorévélable thermiquement stable et avant que l'aspect souhaité ne soit atteint. Cela peut permettre par exemple d'arrêter automatiquement le photomaquillage lorsque l'aspect recherché est atteint. La mesure de la couleur peut se faire par exemple par analyse de la couleur des pixels d'une image formée sur la zone traitée.

Le système de photomaquillage peut être configuré pour réaliser une analyse de régions prédéfinies de l'image et l'on peut contrôler l'irradiation en jouant sur l'intensité de l'irradiation dans différentes zones observées, en fonction de la couleur dans les régions correspondantes. Lorsque l'irradiation s'effectue avec un imageur matriciel adressable, on peut contrôler précisément à cet effet l'irradiation en de multiples pixels de la zone traitée.

L'irradiation peut être constante ou variable. En particulier, elle peut être assez forte pendant un temps donné, dit de « mise à niveau », puis être affaiblie pour une phase d'« affinage ».

Le système de photomaquillage peut être programmé pour délivrer l'irradiation par saccades. Par exemple, une irradiation constante, suivie d'un moment d'arrêt, par exemple d'une durée inférieure ou égale à 30s, et ainsi de suite. L'utilisateur peut arrêter le processus quand il considère qu'il est satisfait.

Dans le cas où l'utilisateur arrête de lui-même l'irradiation puis la recommence, l'irradiation peut être suffisamment lente pour permettre à l'utilisateur de voir évoluer la couleur, l'irradiation évoluant par exemple à une vitesse inférieure ou égale à 3 unités de E par seconde dans l'espace CIE Lab, par exemple d'environ 2 unités de E par seconde.

Lorsque l'intensité de l'irradiation est modulable, le système de photomaquillage peut être doté d'un organe de commande pour agir sur la vitesse et/ou l'amplitude de la diminution ou de l'augmentation de l'irradiation, par exemple un bouton, capteur, joystick, interface à commande vocale ou pavé de contrôle, permettant d'agir sur l'intensité de l'irradiation, notamment en amont de l'imageur.

Selon des exemples de mise en oeuvre de l'invention, l'utilisateur peut arrêter ou ralentir l'irradiation à souhait et ce afin de réfléchir et/ou d'observer le résultat.

On peut prévoir que l'irradiation puisse se faire en fonction de l'exécution par le système de photomaquillage d'un programme d'irradiation et que l'utilisateur puisse soit interrompre ou mettre en pause le programme en cours d'exécution, soit passer d'un programme à un autre. Le programme permettant de définir l'évolution de l'irradiation dans le temps peut être défini ou paramétrable par l'utilisateur, pour régler par exemple les vitesses d'augmentation ou de diminution de l'irradiation.

L'augmentation ou la diminution de l'intensité de l'irradiation peut ne pas provoquer de changement dans la forme ou l'étendue de l'image. Ainsi, on peut utiliser pour moduler l'irradiation des systèmes électriques et/ou optiques agissant sur le flux lumineux réalisé, par exemple au moins un filtre, diaphragme et/ou polariseur, et/ou un variateur de puissance électrique pour commander la source. L'intensité de l'irradiation peut aussi dépendre du niveau de gris des pixels de l'image.

Le système de photomaquillage peut être configuré pour déterminer automatiquement un programme d'illumination progressive en fonction du photomaquillage à réaliser. Par exemple, si le photomaquillage sur une zone donnée consiste à réaliser une couleur assez peu saturée, le système de photomaquillage peut proposer et/ou appliquer un programme commandant une illumination faible. Si le photomaquillage sur une autre zone consiste à réaliser une couleur intense, le système de photomaquillage peut proposer et/ou appliquer un programme consistant à illuminer plus fortement dans un premier temps, puis illuminer ensuite moins fortement, pour permettre à l'utilisateur de régler dans le détail l'achèvement du photomaquillage. L'illumination effectuée dans un premier temps peut l'être avec une fluence au moins double de celle qui est appliquée ensuite.

Pour déterminer l'intensité de l'irradiation, le système de photomaquillage peut se fonder sur un calcul de dose à appliquer pour atteindre le photomaquillage final et appliquer une règle pour en déduire le programme d'illumination. Par exemple, s'il calcule qu'il faudra une dose de X J, il pourra appliquer rapidement 80% de X (en une seconde par exemple) puis appliquer les 20 derniers % au rythme de 5% par seconde, par exemple.

Comme mentionné précédemment, le système de photomaquillage peut être doté d'un dispositif d'acquisition optique lui permettant de mesurer la couleur de la peau ou autres matières kératiniques, soit au début de l'irradiation, soit au cours du photomaquillage. Il peut utiliser ces informations pour calculer ou moduler l'illumination progressive. Par exemple, il peut utiliser ces informations pour identifier le moment où il doit ralentir ou arrêter l'illumination.

Le ou les capteurs du dispositif d'acquisition optique peuvent être monochromes ou polychromes, d'emplacement de mesure monopixel ou multipixel.

Une information représentative de l'avancement du photomaquillage peut être transmise à l'utilisateur de différentes façons, par exemple par affichage d'une valeur représentative de la couleur du photomaquillage en cours ou d'une valeur représentative du degré d'achèvement du processus, par exemple en pourcentage. Il peut aussi y avoir affichage sur l'écran d'une couleur représentant la couleur mesurée.

<u>Retour en arrière</u>

Le système de photomaquillage peut servir à diminuer progressivement l'intensité du photomaquillage, pour

faire disparaître ou régresser une ou plusieurs portions du photomaquillage, grâce à une illumination permettant de ramener le ou les agents photorévélable dans un état non révélé.

De cette façon, l'utilisateur peut revenir en arrière, et mieux régler le rendu final. Le système de photomaquillage est avantageusement réalisé de telle sorte que l'utilisateur puisse arrêter le retour en arrière quand il le désire, relancer le photomaquillage, et ainsi de suite.

La régression du photomaquillage peut être réalisée, pour certains agents photorévélables, par exemple choisis parmi les dyaryléthènes et les fulgides, en remplaçant tout ou partie de l'illumination UV par une illumination visible, par exemple de lumière blanche.

Le système de photomaquillage est configuré préférentiellement pour que cette illumination visible s'étende au moins sur la même surface que l'illumination UV.

<u>Cas d'une composition photorévélable thermiquement stable à agents photorévélables multiples</u>

Lorsque la composition photorévélable thermiquement stable comporte plusieurs agents photorévélables de sensibilités maximales à des longueurs d'ondes respectives différentes, on peut révéler sélectivement un ou plusieurs de ces agents photorévélables en jouant sur la longueur d'onde.

On peut encore utiliser une composition photorévélable thermiquement stable avec plusieurs agents photorévélables différents, à l'état déjà révélé, et s'effaçant le mieux à des longueurs d'onde respectives différentes. Le cas échéant, ces agents photorévélables peuvent être révélés par une même lumière UV, mais ont des vitesses d'effacement dans le visible qui varient en fonction de la longueur d'onde, de telle sorte qu'en choisissant celle-ci on peut privilégier l'effacement d'un agent photorévélable plutôt qu'un autre. De même que ci-dessus, lorsque les agents photorévélables sont révélables par une lumière UV, ceux-ci peuvent se révéler avec des vitesses différentes en fonction de la longueur d'onde dans le domaine UV, et en jouant sur cette longueur d'onde UV on peut privilégier la révélation d'un agent photorévélable par rapport aux autres.

<u>Simulation de l'évolution du photomaquillage</u>

Dans un exemple de mise en oeuvre de l'invention, le système de photomaquillage est doté, en plus ou en remplacement d'un système de visualisation de l'évolution du photomaquillage, d'un système de simulation de l'évolution du photomaquillage.

L'utilisateur peut alors observer, avant et/ou pendant le photomaquillage, cette simulation, et se fonder dessus pour décider de ralentir ou d'arrêter le photomaquillage, voir de revenir en arrière.

Le système de photomaquillage peut être configuré pour permettre de simuler le résultat du photomaquillage après l'application de la composition photorévélable thermiquement stable et avant que l'aspect souhaité ne soit atteint. L'avancement de la simulation peut être lié à l'avancement de l'irradiation sur la zone à traiter, qu'elle serve à révéler la composition photorévélable thermiquement stable ou au contraire à l'effacer. Une simulation de l'évolution de l'aspect du photomaquillage peut être affichée sur un écran et/ou projetée sur la zone traitée, lorsque l'irradiateur utilisé le permet. La simulation peut être projetée dans une lumière ne provoquant pas la révélation de la composition photorévélable thermiquement stable, ni son effacement, au moins sur une courte période, le temps pour l'observateur de décider de la suite du traitement.

<u>Utilisation d'outils</u>

Lorsque le système de photomaquillage comporte un imageur électronique, ce dernier peut être commandé en fonction d'un outil manipulé par l'utilisateur, le calculateur pouvant modifier l'image projetée et/ou l'intensité de l'irradiation en fonction d'un déplacement de l'outil. Ce dernier peut comporter une partie à positionner devant ou sur la zone à traiter ou devant ou sur un écran de visualisation de la zone à traiter. L'outil peut aussi commander le déplacement d'un pointeur sur un écran de visualisation de la zone à traiter ou au sein de l'image formée sur la zone à traiter.

Le système de photomaquillage peut être configuré pour permettre à l'utilisateur de commander des zones particulières qu'il veut traiter d'une irradiation progressive, et utiliser à cet effet un moyen de visualisation, qui peut comporter par exemple un écran tactile par lequel l'utilisateur peut commander, en appuyant sur une région particulière de l'écran, la progression de l'irradiation. De façon encore plus préférée, l'écran tactile est sensible à l'intensité de la pression exercée par l'utilisateur. Le système de photomaquillage peut analyser la pression exercée sur l'écran et traduire cette pression en intensité de photomaquillage, en contrôlant l'intensité de la lumière et/ou la durée de l'irradiation sur la région correspondante de la zone à traiter. Ainsi, par exemple, une plus forte pression exercée sur l'écran tactile se traduira en une intensité colorielle accrue.

Dans un exemple de mise en oeuvre de l'invention, le système de photomaquillage peut détecter un outil placé sur ou devant l'écran tactile, avec lequel utilisateur peut agir sur le photomaquillage.

Par exemple, l'utilisateur peut disposer de plusieurs outils munis chacun d'un moyen d'identification, par exemple un code à barres ou une puce RFID, de façon à pouvoir être identifié par le système de photomaquillage. Lorsque l'utilisateur prend un outil particulier, celui-ci est reconnu et chaque outil peut être associé par le système de photomaquillage à un type de maquillage particulier.

Par exemple, l'utilisateur dispose de plusieurs outils correspondant à des traits de maquillage plus ou moins épais et/ou à des intensités de couleur plus ou moins grandes, voire des couleurs de maquillage différentes. L'utilisateur prend l'outil qu'il souhaite et peut le déplacer sur l'image du moyen de visualisation pour faire évoluer le maquillage. Une simulation du maquillage peut apparaître sur l'écran de visualisation et après une validation éventuelle par l'utilisateur, le maquillage apparaissant sur le moyen de visualisation peut être automatiquement réalisé par photomaquillage en commandant l'irradiateur.

Ajustement et/ou modification du contenu de l'image

Dans un exemple de mise en oeuvre de l'invention, on révèle une couche de photomaquillage avec la même image de simulation que celle projetée en lumière visible. Pour cela, le système de photomaquillage peut être configuré pour envoyer une image sur la zone à traiter, par exemple le visage, représentant la simulation, laissant à l'utilisateur la possibilité de caler cette image sur le visage, voire de la modifier. Puis, une fois l'image correctement ajustée et définie, on envoie sur la même zone, par le biais de la même optique, ou par le biais d'une optique parallèle, une image qui peut ne différer de la précédente qu'en ce qu'elle est formée avec une lumière UV ou dans le proche UV. En particulier, le masque, négatif ou matrice de pixels adressables utilisé pour définir l'image peut ne pas avoir été modifié lors du changement d'illuminant de la lumière visible à la lumière UV. Pour ce faire, on peut utiliser un système de photomaquillage doté d'une source UV et d'une source visible, avec un ou deux imageurs.

Si l'image est obtenue à partir d'une diapositive, cette dernière peut être prévue pour permettre de réaliser une image avec la source visible et une image avec la source UV. Pour cela, on peut réaliser la diapositive avec une matière filtrant à la fois la lumière UV et la lumière visible.

Si l'image est obtenue avec au moins une matrice de pixels adressables d'un imageur électronique, plusieurs configurations sont possibles :

- deux sources lumineuses, à savoir une source UV et une source visible, et une seule matrice ;
- deux sources lumineuses, respectivement UV et visible, et deux matrices, respectivement UV et visible. Les images sont rassemblées, par exemple avec un prisme en X ou projetées depuis des emplacements situés de part et d'autre de la zone traitée ;
- une source lumineuse émettant à la fois dans l'UV et dans le visible et un imageur UV ou visible, avec par exemple un miroir dichroïque, un miroir mobile ou un filtre pour sélectionner le rayonnement sortant ;
- une source lumineuse émettant à la fois dans l'UV et dans le visible et deux matrices respectivement pour l'UV et pour le visible.

L'image projetée peut se restreindre à un contour à ou quelques points de référence. Le photomaquillage réalisé ensuite peut s'inscrire entre ces points ou ce contour.

Par exemple, dans le cas du traitement des lèvres, on peut se servir de deux points de référence. L'utilisateur applique une couche de composition photorévélable thermiquement stable. On projette les deux points dans une longueur d'onde visible, incapable de produire un effet de photomaquillage, par exemple avec une longueur d'onde comprise entre 450 et 800 nm, et préférentiellement de 500 nm à 700 nm. L'utilisateur positionne ces deux points sur les deux commissures des lèvres. Une fois le positionnement réalisé, l'irradiation UV forme une image de lèvres comprise entre ces deux points.

Pendant l'illumination UV, l'image visible peut être coupée, réduite ou limitée à des points de référence, ou laissée dans le même état que pendant la phase de projection dans le visible.

Utilisation du photomaquillage pour réaliser sur le visage un patron de traitement puis s'en servir pour traiter la zone

On applique sur la zone à traiter un agent photorévélable, par exemple choisi parmi les diaryléthènes ou fulgides, ayant une stabilité thermique suffisante pour que l'image qui est réalisée puisse se maintenir au moins 20 secondes, et de préférence au moins 1 minute. Le patron peut être une image comportant des points et/ou des lignes de repérage, comme illustré à la figure 11, où l'on voit des lignes délimitant des zones A, B et C réalisées par photomaquillage. Le cas échéant, on réalise par photomaquillage un signe, par exemple alphanumérique, renseignant l'utilisateur sur le produit et/ou l'applicateur à utiliser dans cette zone.

L'utilisateur peut réaliser le maquillage ou autre traitement en fonction du patron ainsi révélé. Pour cela, il peut utiliser des outils de maquillage classiques. L'utilisateur peut aussi utiliser le patron réalisé par photomaquillage pour

appliquer des produits de soin.

Par exemple, on réalise un diagnostic des zones nécessitant un soin particulier, par exemple par analyse d'image et/ou à l'aide d'un ou plusieurs capteurs sensibles à l'état cutané par exemple, et l'on réalise une cartographie du traitement à effectuer, qui peut être mémorisée. Puis on utilise le système de photomaquillage pour faire apparaître un patron sur le visage, où seront révélées de façon colorée au moins temporairement les zones nécessitant le soin. L'utilisateur peut alors appliquer sur les zones ainsi mises en exergue le ou les produits de soin.

Positionnement des points de référence sur le visage, puis utilisation d'un appareil reconnaissant ces points et s'en servant pour réaliser le maquillage

L'utilisateur peut positionner des points de référence sur le visage par photomaquillage.

Une fois les points de référence placés sur la zone à traiter, on peut utiliser un appareil doté de moyens de lecture des points de référence, et de préférence capable également de les interpréter si nécessaire. L'appareil peut être doté, dans un exemple de mise en oeuvre, d'un capteur multipixel, d'un éclairage interne, et d'un moyen d'application, par exemple une tête d'impression à jet d'encre.

Les points représentent par exemple une ligne. L'utilisateur déplace sur la peau l'appareil, qui est configuré pour en analyser optiquement la surface. Par exemple, en supposant que la zone à traiter est délimitée par un contour fermé, quand l'appareil détermine qu'il traverse une première fois une ligne, il se met à déposer une matière colorée, puis quand il traverse une seconde fois une ligne, il arrête de déposer la matière colorée.

Les points peuvent représenter, quand ils sont reliés, une forme. L'appareil peut être doté de moyens permettant de reconnaitre ces points et la forme correspondante. L'appareil peut déposer une matière colorée de manière à réaliser cette forme. Pour cela, l'appareil peut partir d'un modèle qu'il fait coïncider avec les points en réalisant des rectifications géométriques.

Le dépôt de matière colorée peut définir une courbe ou une surface inscrite dans une courbe.

La couverture de la ligne et/ou de la surface peut être homogène, aléatoirement hétérogène ou géométriquement hétérogène, c'est-à-dire comportant un motif répétitif.

Les points présents sur la zone à traiter peuvent définir un code. L'appareil peut être doté de moyens permettant d'interpréter le code correspondant à ces points, et appliquer un produit dont la sélection et/ou le mode d'application s'effectue en fonction du code reconnu.

Par exemple, certains points sont formés avec un premier motif et d'autres avec un motif différent. L'appareil applique deux produits différents ou un même produit à des concentrations différentes en fonction du motif détecté.

**Composition photoprotectrice**

Comme évoqué plus haut, une composition photoprotectrice peut être appliquée sur la composition photorévélable thermiquement stable une fois l'aspect souhaité atteint. Cette composition photoprotectrice peut faire écran au rayonnement UV lorsque l'irradiation permettant de révéler la composition photorévélable thermiquement stable est une irradiation UV.

La composition photoprotectrice peut aussi, le cas échéant, faire écran à au moins une longueur d'onde prédéfinie dans le visible, en vue de limiter le risque d'effacement d'un agent photorévélable, le cas échéant.

Un ou plusieurs agents optiques assurant à la composition photoprotectrice un pouvoir filtrant F du rayonnement solaire (290 à 400 nm) allant de 2 à 20, de préférence de 4 à 10, peuvent être utilisés.

La composition photoprotectrice peut être, le cas échéant, un produit brillantant, gras ou émollient, antibrillantant, un rose à joue, un blush, un vernis ou un produit d'apprêt.

**Agents optiques**

Agents optiques faisant écran au rayonnement servant à la révélation, notamment UV ou proche UV.

Le ou les agents optiques précités peuvent être choisis parmi les filtres et les particules diffusantes ou autres agents limitant la transmission des UV, notamment les UVA et/ou les UVB.

Ce ou ces agents optiques peuvent être choisis parmi les filtres inorganiques, notamment sous forme particulaire et de taille nanométrique, et les filtres organiques.

Le ou les agents optiques peuvent être hydrophiles ou lipophiles.

Les filtres organiques peuvent être choisis parmi les dérivés anthranilates, cinnamiques, les dérivés salicyliques, les dérivés du camphre, les dérivés du benzimidazoles, du benzotriazole, les dérivés du benzalmalonate, les imidazolines,

les bisbenzoazolyle, les dérivés de benzoxazole, les dérivés de triazine, les dérivés de benzophénones, les dérivés de dibenzoylméthane, les dérivés de béta diphénylacrylate, les dérivés de p-aminobenzoique, des polymères filtres et silicones filtres décrits dans la demande WO 93/04665, les dimères dérivés d'alpha alkylstyrène, les 4,4-diarylbutadiène et leurs mélanges.

Les filtres hydrophiles peuvent être choisis parmi ceux décrits dans la demande EP A 678 292, par exemple le 3-benzylidene 2-camphre, notamment le Mexoryl SX®.

Parmi les filtres lipophiles, on peut citer les dérivés du dibenzoylméthane, décrits dans les publications FR 2 326 405, FR 2 440 933, EP 0 114 607, le Parsol® 1789 de la société Givaudan, l'Eusolex de la société Merck. On peut aussi citer l'a-cyano-b, b-diphénylacrylate de 2 ethylhexyle, appelé octocrylène et disponible sous le nom commercial de Uvinul N 539 auprès de la société BASF.

On peut aussi citer le p-méthylbenzylidène camphre, vendu sous le nom commercial d'Eusolex EX 6300 de la société Merck.

On peut encore utiliser comme actif optique un filtre choisi parmi benzophenone-3 (oxybenzone), benzophenone-4 (sulisobenzone), benzophenone-8 (dioxybenzone), Bis-ethylhexyloxyphenol methoxyphenyl triazine (BEMT ou Tinosorb S), Diethylamino Hydroxybenzoyl Hexyl Benzoate (Uvinul +), Ethylhexyl Methoxycinnamate, Ethylhexyl Salicylate, ethylhexyl triazone, methyl anthranilate (meradimate), (4-)methyl-benzylidene camphre (Parsol 5000), Methylene Bis-Benzotriazolyl Tetramethylbutylphenol (Tinosorb M), acide para-aminobenzoique (PABA), acide Phenylbenzimidazole Sulfonic (Ensulizole), polysilicone 15 (Parsol SLX), triethano lamine salicylate.

On peut encore utiliser comme agent optique des particules diffusantes telles que les nanopigments d'oxyde de titane ou de zinc, utilisables comme filtre, avec divers traitements de surface selon le milieu choisi. Les nanopigments ont typiquement une taille moyenne de 5 à 1000nm.

La concentration massique totale en de tels agent(s) optique(s) peut aller de 0,001% à 30%, voire plus dans le cas des formules sèches ou presque sèches, par rapport au poids de la composition photoprotectrice, avant application.

De préférence, on utilise au sein de la composition des filtres ou associations permettant de faire écran au rayonnement dans la plage allant de 320 à 400 nm, et de préférence allant de 320 à 420 nm.

<u>Actifs optiques destinés à limiter la propagation de la lumière visible et infrarouge vers le ou les agents photorévélables</u>

Si les agents optiques faisant écran au rayonnement UV permettent aux zones non révélées d'être protégées, dans le cas d'une composition photorévélable thermiquement stable révélable par irradiation UV, on peut aussi appliquer sur la composition photorévélable thermiquement stable un ou plusieurs agents optiques filtrant dans le visible, pour protéger les zones révélées, et l'on peut avoir intérêt à combiner les deux, à savoir filtration dans l'UV et filtration dans le visible.

De nombreux colorants ou pigments sont utilisables. En particulier, on préfère les colorants de couleur proche de celle de la peau, par exemple les colorants jaunes, oranges ou les mélanges permettant de réaliser des teintes jaune, orange, ocre, marron ou brunes ou bien des colorants rouges que l'on emploiera de façon préférentielle soit en petite quantité, soit en mélange avec un diffusant blanc ou jaune par exemple, pour donner à la teinte un aspect pastel tel que rose ou rose beige. On préfère les teintes de colorants un peu roses pour les peaux roses, les teintes un peu jaunes pour les peaux roses et les teintes marron ou brun pour les peaux dites noires.

Les colorants peuvent présenter soit seuls, soit en mélange, une chromaticité proche de celle de la peau. Ils sont préférentiellement de chroma C* (dans le système HVC*) inférieur à 40.

Le ou les colorants peuvent être choisis parmi :

- les pigments jaunes codifiés dans le Color Index sous les références CI 11680, 11710, 15985, 19140, 20040, 21100, 21108, 47000, 47005,
- les pigments oranges codifiés dans le Color Index sous les références CI 11725, 15510, 45370, 71105, et
- les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470.

On peut utiliser au sein de la composition photoprotectrice des pâtes pigmentaires de pigment organique telles que les produits vendus par la société HOECHST sous les noms :

JAUNE COSMENYL IOG : Pigment 5 t YELLOW 3 (CI 11710)
JAUNE COSMENYL G : Pigment YELLOW 1 (CI 11680)
ORANGE COSMENYL GR : Pigment ORANGE 43 (CI 71105)

On peut utiliser des laques et, en particulier, celles connues sous les dénominations D & C Red 21 (CI 45 380),

D & C Orange 5 (CI 45370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Yellow 1 O (CI 77 002).

Les colorants peuvent être ioniques ou neutres.

Sont particulièrement appréciés les colorants et pigments naturels car ils se combinent bien aux teints naturels et pour certains perdent au cours du temps leur couleur. Ce sont par exemple des extraits de plantes ou des molécules naturelles reproduites artificiellement, étant choisis par exemple parmi la mélanine, les anthocyanes, polyphénols, porphyrines et curcumine.

Il s'agit par exemple des pigments obtenus par polymérisation oxydante de dérivés indoliques et/ou phénoliques, tels qu'ils sont décrits dans la publication FR 2 679771.

Sont particulièrement appréciés les colorants et pigments présentant une ionicité complémentaire des filtres UV, par exemple les colorants à fonction anionique, comme certains colorants alimentaires et les filtres cationiques.

On peut aussi utiliser des filtres IR ou des composés qui, par réaction, vont se colorer, par exemple la DHA.

On peut utiliser un colorant évolutif, par exemple un colorant dont la couleur se révèle au cours du temps et si possible lentement, par exemple la DHA ou les polyphénols, aptes à se colorer progressivement au contact de l'air. Cela permet de développer progressivement le pouvoir filtrant de la composition photoprotectrice.

Agents photorévélables thermiquement instables

On peut utiliser comme agent optique dans la composition photoprotectrice un agent de coloration photorévélable thermiquement instable. Celui-ci ne sert pas à créer le photomaquillage mais à le protéger en cas d'exposition lumineuse trop intense, par exemple en cas d'ensoleillement très fort ou d'un effet artificiel tel que l'illumination utilisée sur les plateaux de télévision, certains traitements médicaux, certains traitements cosmétiques tels que les cabines UV par exemple, des flashs photographiques ou certains endroits festifs.

L'agent photorévélable thermiquement instable va prendre sa couleur le temps de l'illumination très intense et, d'une certaine façon, peut limiter la visibilité du photomaquillage sous-jacent. Cependant, comme l'agent photorévélable thermiquement instable reprend vite sa forme incolore après arrêt de l'illumination très intense, ce phénomène est passager.

De préférence, on utilise des agents photorévélables thermiquement instables, perdant au moins la moitié de leur couleur en 60 secondes à 25°C dans l'obscurité. En particulier, on préfère les agents photorévélables thermiquement instables inorganiques.

Agents optiques capables de réfléchir la lumière incidente

On peut utiliser comme agent optique, notamment pour atténuer les UV ou la lumière visible, un agent optique formant miroir métallique ou un agent optique à base de structure multicouche interférentielle ou de réseau de diffraction.

Comme agents optiques utilisables seuls ou en complément des agents optiques énumérés ci-dessus, on peut utiliser des agents optiques capables de réfléchir la lumière incidente. La réflexion se produit à l'interface entre la couche réfléchissante et le milieu de propagation de l'onde lumineuse. Le matériau formant la couche réfléchissante peut présenter un indice de réfraction supérieur à 1,5, si possible supérieur à 1,8.

L'agent optique peut contenir ou être formé d'un métal. Par exemple, on forme en appliquant la composition photoprotectrice une couche d'argent par le biais de la réduction d'un sel d'argent ou par le biais de l'application d'une dispersion de nanoparticules d'argent.

Le taux de réflexion de la composition photoprotectrice peut être supérieur à 5%, et si possible supérieur à 10%. De préférence, il est inférieur à 50%, pour ne pas entacher le résultat du photomaquillage. Par exemple, la composition photoprotectrice peut comporter une dispersion soit aqueuse, soit éthanolique, de nanoparticules d'argent, par exemple celles de la société Nippon Paint qui présentent une taille allant de 10 à 60 nm selon les échantillons et sont stabilisées par un système polymérique. Cette stabilisation n'empêche pas, au moment du séchage, aux particules de se mettre en contact, et par le biais de ces contacts, d'assurer une conductivité suffisante pour donner au matériau final un pouvoir réfléchissant proche de celui obtenu avec un miroir d'argent.

On peut utiliser un agent optique comportant une structure multicouche interférentielle.

Cette structure interférentielle peut filtrer la lumière par un phénomène d'interférences destructives entre les ondes lumineuses réfléchies par les différentes couches de la structure.

La structure multicouche est choisie préférentiellement de manière à présenter un facteur de transmission élevé dans le visible, de manière à ne pas produire de couleur marquée dans le visible et à présenter la transparence recherchée.

La structure multicouche peut comporter une alternance de couches de bas et hauts indices de réfraction. La différence d'indice de réfraction entre les couches de haut et de bas indice est par exemple supérieure ou égale à 0,1,

mieux 0,15, encore mieux 0,6.

Le nombre de couches de l'alternance précitée est par exemple d'au moins 2, mieux 4 ou 6, voire au moins 12, ce qui facilite la réalisation d'une structure peu sensible à l'incidence de la lumière et présentant la sélectivité requise. La structure multicouche peut éventuellement être symétrique, et permettre une filtration de lumière incidente quelle que soit la face principale d'entrée de la lumière dans la structure, le cas échéant.

Le matériau à indice de réfraction élevé peut être minéral, par exemple du dioxyde de titane sous forme anatase ou rutile, un oxyde de fer, du dioxyde de zirconium, de l'oxyde de zinc, du sulfure de zinc, de l'oxychlorure de bismuth, et leurs mélanges, ou organique, étant par exemple choisi parmi : PEEK (polyétheréthercétone), polyimide, PVN (Poly (2-vinylnaphtalène)), PVK (Poly(N-vinyl carbazole)), PF (résine phénolformaldéhyde), PSU (résine polysulfone), PaMes (poly(alpha-méthylstyrène)), PVDC, (Poly(vinylidène chloride)), MeOS (Poly(4-méthoxystyrène)), PS (polystyrène), BPA, (bisphénol-A polycarbonate), PC (résine polycarbonate), PVB (Poly(vinyl benozoate)), PET (poly(éthylènetéréphthalate)), PDAP (poly(diallyl phthalate)), PPhMA (poly(phénylméthacrylate)), SAN (styrène/acrylonitrile copolymère), HDPE (polyéthylène, haute densité), PVC (poly(vinyl chloride)), NYLON®, POM (poly(oxyméthylène) ou polyformaldéhyde), PMA (poly(méthyl acrylate)), etc., et leurs mélanges.

Le matériau à bas indice de réfraction peut être minéral, étant par exemple choisi parmi le dioxyde de silicium, le fluorure de magnésium, l'oxyde d'aluminium, et leurs mélanges, ou organique, étant par exemple choisi parmi des polymères tels que le polyméthylméthacrylate ou le polystyrène, le polyuréthane, et leurs mélanges.

Pour réaliser les particules interférentielles à structure multicouche, l'homme du métier pourra notamment se référer aux nombreuses publications qui traitent du dépôt en couches minces, par exemple l'article Overcoated Microspheres for Specific Optical Powers de la revenue Applied Optics, Vol. 41, n° 6 du 01/06/2002, ici incorporé par référence, et aux brevets de la société FLEXPRODUCTS. L'agent optique peut comporter une structure diffractante, et par exemple au moins un réseau de diffraction, lequel peut être un réseau comportant un motif de surface se répétant sensiblement de manière à diffracter la lumière.

La période du réseau et éventuellement la profondeur de celui-ci déterminent entre autres les propriétés de diffraction du réseau. Le rapport cyclique du réseau de diffraction peut être choisi égal à l'unité.

De façon préférentielle, la période du réseau de diffraction, dans au moins une direction, est avantageusement suffisamment faible pour réduire le risque de création d'effets colorés dans la composition photoprotectrice. La période du réseau est ainsi avantageusement choisie de manière à ne pas diffracter la lumière dans le domaine visible, notamment dans la plage allant de 400 nm à 780 nm.

La période maximale du réseau qui permet d'éviter d'avoir des ordres de diffraction dans le visible peut être déterminée au moins de manière approximative par la relation :

$$n_1 \sin\theta + \frac{m\lambda}{\Lambda} = n_2 \sin\Psi,$$

où θ est l'angle d'incidence mesuré par rapport à la normale au plan du réseau, Ψl'angle de transmission, Λ la période du réseau, m l'ordre de diffraction et $n_1$ et $n_2$ les indices de réfraction des milieux d'incidence et de transmission, respectivement. $n_1$ et $n_2$ peuvent être pris égaux à 1,5 en première approximation. Pour θ = 0°, la période maximale est de $\lambda/n_1$ = 400/1,5 soit 267 nm environ. Sans limitation sur l'angle d'incidence, la période est moitié moindre. On choisira donc, de préférence, une période pour le réseau inférieure ou égale à 270 nm, mieux inférieure ou égale à 140 nm.

La profondeur *d* du réseau et sa période Λ peuvent être sélectionnées par des essais successifs de manière à obtenir par exemple une transmission minimale dans les UVA. Le calcul des caractéristiques du réseau peut s'effectuer par calcul vectoriel en utilisant par exemple le logiciel GSOLVER de la société GRATING SOLVER

DEVELOPMENT COMPANY.

La ou les différentes couches utilisées pour réaliser le ou les réseaux de diffraction peuvent éventuellement être déposées sur un substrat de nature organique ou minérale, lequel peut être utilisé tel que ou subir ensuite un traitement de dissolution.

Ainsi, la structure du ou des réseaux peut être gravée soit dans la masse d'un matériau soit après le dépôt d'un matériau sur un substrat organique ou minéral de forme sphérique ou lamellaire.

La gravure peut être réalisée de façon à ce que la diffraction de la lumière dans la partie visible soit minimale, pour réduire les effets colorés. La périodicité de la gravure et son épaisseur déterminent l'efficacité du système à atténuer le rayonnement UV.

L'agent de filtration interférentiel peut éventuellement comporter deux réseaux de diffraction s'étendant dans

des directions non parallèles, par exemple deux directions sensiblement perpendiculaires, ce qui peut notamment permettre d'augmenter l'absorption dans les UV d'une lumière incidente polarisée circulairement et de réduire la dépendance des performances de filtration vis-à-vis de l'angle d'incidence.

Les deux réseaux de diffraction peuvent avoir des périodes $\Lambda_1$ et $\Lambda_2$ sensiblement égales, notamment toutes deux inférieures ou égales à 270 nm, mieux à 140 nm.

Les deux réseaux de diffraction peuvent présenter également des profondeurs sensiblement égales, lorsqu'ils présentent un relief en surface et que ce relief permet de créer la variation périodique d'indice du réseau.

La période du réseau peut être constante ou variable et la profondeur constante ou variable. Le réseau peut s'étendre selon une direction rectiligne ou curviligne.

Le réseau de diffraction peut comporter une superposition de couches ayant des indices de réfraction différents. Le réseau de diffraction peut être réalisé au moins partiellement dans un matériau diélectrique.

Les motifs du ou des réseaux peuvent être divers et par exemple présenter, en coupe, des créneaux rectangulaires, triangulaires, des ondulations sinusoïdales ou des créneaux en escalier.

La structure diffractante peut être formée au moins sur une portion d'une face principale de la particule et de préférence sur les deux faces principales de la particule.

La structure diffractante peut comporter une couche de protection recouvrant le ou les réseaux et non diffractante.

On peut également citer les pigments à effet interférentiel non fixés sur un substrat comme les cristaux liquides (Helicones HC de la société Wacker), et les paillettes holographiques interférentielles (Geometric Pigments ou Spectra f/x de la société Spectratek).

La composition peut comporter un mélange d'éléments interférentiels filtrant les UVA et/ou les UVB, par exemple des particules présentant des réseaux de diffraction ayant des périodes et/ou des profondeurs différentes.

<u>Agents optiques capables de transformer la longueur d'onde de la lumière incidente</u>

La composition photoprotectrice peut comporter un composé fluorescent.

Par composé « fluorescent », on entend un composé qui absorbe la lumière du spectre ultraviolet, et éventuellement du visible et qui transforme l'énergie absorbée en lumière fluorescente de plus grande longueur d'onde, émise dans la partie ultraviolet ou visible du spectre.

Il peut s'agit d'azurants optiques, qui peuvent être transparents et incolores, n'absorbent pas dans la lumière visible mais uniquement dans les UV et transforment l'énergie absorbée en lumière fluorescente de plus grande longueur d'onde, par exemple plus longue de 20 nm, mieux 50 nm, voire 100 nm, émise dans la partie visible du spectre; l'impression de couleur générée par ces azurants peut alors uniquement engendrée par la lumière purement fluorescente à prédominante bleue, de longueurs d'onde allant de 400 à 500 nm.

Ces composés peuvent être en solution ou particulaires.

Le composé fluorescent peut être un dicetopyrrolopyrrole de formule :

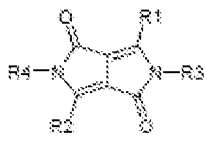

dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène, ce radical alkyle pouvant être interrompu par un hétéroatome ;

Le composé fluorescent peut être un naphtalimide, de formule :

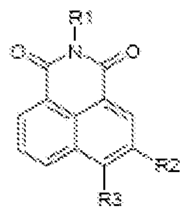

où

R1, R2, R3, indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino; un groupement benzoylalkyle (C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène, ce 5 radical alkyle pouvant être interrompu par un hétéroatome ; les substituants R1 , R2, R3 peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4) ammonioalkyl(C1-C4) ;

Le composé fluorescent peut être un dérivé stylbénique comme :

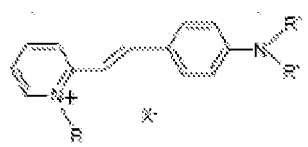

formule dans laquelle R représente un radical méthyle ou éthyle; R' représente un radical méthyle, X- un anion du type chlorure, iodure, sulfate, méthosulfate, acétate, perchlorate. A titre d'exemple de composé de ce type on peut citer le Photosensitiving Dye NK-557 commercialisé par la société UBICHEM, pour lequel R représente un radical éthyle, R' un radical méthyle et X- un iodure.

Le composé fluorescent peut être un dérivé méthynique comme :

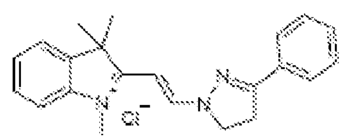

ou un dérivé oxazine ou thiazine de formule générale :

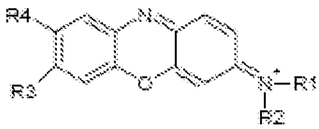

On peut citer aussi les dérivés dicyanopyrazines (de la société Nippon Paint), les dérivés de naphtolactames, les dérivés azalactones, les rhodamines, les dérivés de xanthènes.

On peut également utiliser des pigments ou particules minérales (MgO, TiO2, ZnO, Ca(OH)2...) ou organiques (latex...) comprenant en leur coeur ou sur leur surface de tels composés.

Le composé fluorescent peut aussi être un composé semi-conducteur qui, par exemple sous la forme de petites particules, appelées quantumdots, présente un effet fluorescent.

Les quantum dots sont des nanoparticules semi-conductrices luminescentes capables d'émettre, sous excitation lumineuse, un rayonnement présentant une longueur d'onde comprise entre 400 nm et 700 nm. Ces nanoparticules peuvent être fabriquées selon les procédés décrits par exemple dans les brevets US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

Des composés fluorescents préférés sont ceux émettant les couleurs orangée et jaune, par exemple.

De préférence, le ou les composés fluorescents utilisés en tant qu'agents optiques dans l'invention ont un maximum de réflectance dans la gamme de longueurs d'onde allant de 500 à 650 nm, et de préférence dans la gamme de longueurs d'onde allant de 550 à 620 nanomètres.

De tels composés fluorescents sont par exemple ceux appartenant aux familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges.

Plus particulièrement, on peut citer parmi :

- le Jaune Brilliant B6GL commercialisé par la société SANDOZ et de structure suivante :

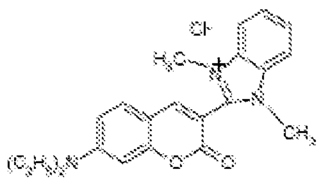

- le Basic Yellow 2, ou Auramine O commercialisé par les sociétés PROLABO, ALDRICH ou CARLO ERBA et de structure suivante

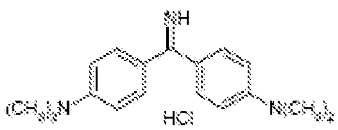

Les composés fluorescents utilisés peuvent être des aminophényl ethenyl aryl, où aryl est un pyridinium, substitué ou non, ou un autre groupe cationique tel que des imidizoliniums substitués ou non.

Par exemple, on peut utiliser un composé fluorescent tel que le 2-[2-(4-dialkylamino)phényl éthényl]-1 alkyl

pyridinium, dans lequel le radical alkyle du noyau pyridinium représente un radical méthyle, éthyle, celui du noyau benzénique représente un radical méthyle.

Un agent optique peut contenir sur une même molécule, plusieurs groupes fluorescents. A titre d'exemple, ce sont des dimères tels que :

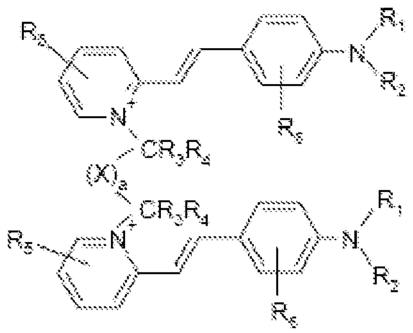

$n Y^-$

où R1, R2, identiques ou différents, représentent

- un atome d'hydrogène ;
- un radical alkyle, linéaire 5 ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
- un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène.

R1 et R2 peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;

R1 ou R2 peut éventuellement être engagé dans un hétérocycle comprenant l'atome d'azote et l'un des atomes de carbone du groupement phényle portant ledit atome d'azote ;

R3, R4, identiques ou non, représentent un atome d'hydrogène, un radical alkyle comprenant 1 à 4 atomes de carbone.

R5, identiques ou non, représentent un atome d'hydrogène, un atome d'halogène, un radical alkyle linéaire ou ramifié comprenant 1 à 4 atomes de carbone éventuellement interrompu par au moins un hétéroatome ;

R6, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou un groupement portant au moins un hétéroatome et/ou substitué par au moins un atome d'halogène ;

X représente :

- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène

ou par au moins un radical alkyle comprenant 1 à 10 atomes de carbone éventuellement substitué et/ou interrompu par au moins un hétéroatome et/ou groupement portant au moins un hétéroatome;

- un radical dicarbonyle ;
- le groupement X pouvant porter une ou plusieurs charges cationiques ;
- a étant égal à 0 ou 1 ;

Y-, identiques ou non, représentent un anion organique ou minéral ;
n étant un nombre entier au moins égal à 2 et au plus égal au nombre de charges cationiques présentes dans le composé fluorescent.

D'autres dimères sont possibles, comme par exemple ceux où le point de rattachement est réalisé entre les deux groupes non cationiques, ou par exemple ceux où le groupe pyridinium est remplacé par un autre groupe arylcationique tel qu'un imidazolinium.

La famille des dicyanopyrazines peut aussi fournir des composés fluorescents dans les orangés et intéressants pour l'invention.

Les pigments fluorescents dans les orangés sont aussi utilisables. Par exemple, le pigment Sunbrite-SG2515 Yellow orange de la société SunChemical.

**Application de la composition photoprotectrice**

L'utilisateur peut appliquer la composition photoprotectrice sur l'ensemble de la zone traitée, en dépassant largement la couche de composition photorévélable thermiquement stable ou au contraire de façon localisée sur certaines zones seulement, comme illustré à la figure 12. Sur cette figure, le photomaquillage a été réalisé avec une composition photorévélable thermiquement stable PC recouverte largement par la composition photoprotectrice PP. La composition photoprotectrice peut par exemple être localisée sur les bords de la zone revêtue de la composition photorévélable thermiquement stable, entourant ainsi les motifs du photomaquillage dans le cas où le photomaquillage est moins étendu que la couche de composition photorévélable thermiquement stable.

La couche de composition photoprotectrice peut aussi prendre la forme d'un film souple à coller sur les matières kératiniques, par exemple la peau. La matière du film peut agir comme agent optique et/ou le film peut contenir au moins un agent optique dispersé dans la matière du film. Le film peut encore porter un revêtement contenant l'agent optique, par exemple sous la forme d'une impression ou d'une structure multicouche interférentielle.

L'utilisateur peut appliquer un tel film sur l'ensemble de la couche de composition photorévélable thermiquement stable ou peut éventuellement découper le film pour couvrir seulement des zones non révélées, en ne couvrant pas les zones révélées.

On peut utiliser un système de découpage automatique, qui partant du contenu du photomaquillage, par exemple de son contour, va découper le film de protection à la forme adaptée. L'utilisateur placera alors le film de protection ainsi découpé sur les zones non révélées.

Dans un autre exemple de mise en oeuvre de l'invention, la composition photoprotectrice est déposée par transfert, en appliquant une feuille de support portant au moins un agent optique sur la zone à traiter. L'utilisateur peut amener la feuille en contact avec les matières kératiniques revêtues de la composition photorévélable thermiquement stable, puis par frottement ou par d'autres moyens tels que la chaleur ou l'utilisation d'un solvant, provoquer le transfert du ou des agents optiques sur la couche de composition photorévélable thermiquement stable.

Dans un exemple de mise en oeuvre de l'invention, la couche de composition photoprotectrice est réversible, c'est-à-dire que l'utilisateur peut l'enlever sans ôter la première couche de composition photorévélable thermiquement stable.

Pour cela, on peut formuler la première couche de façon qu'elle soit résistante à l'eau ou à un mélange d'eau et de tensioactif et formuler la seconde couche de façon qu'elle soit non résistante à l'eau ou à un mélange d'eau et de tensioactif.

On peut aussi réaliser une seconde couche pelable. Pour cela, on peut utiliser une seconde couche formant, avant ou après application sur la première couche, un revêtement cohésif. La seconde couche, lorsqu'elle est pelable, comporte par exemple un matériau élastomérique.

Dans un exemple de mise en oeuvre de l'invention, la deuxième couche est peu adhérente à la première couche, par exemple grâce à l'emploi dans la composition photorévélable thermiquement stable de composés de basse tension de surface tels que des composés siliconés ou fluorés. Dans un autre exemple de mise en oeuvre de l'invention, on intercale entre la première et la seconde couche une couche intermédiaire antiadhésion, facilitant l'enlèvement de la couche de composition photoprotectrice.

La couche de composition photoprotectrice peut avoir, notamment lorsqu'elle est réversible, un pouvoir filtrant F très important, par exemple supérieur ou égal à 20.

On peut déposer une seule couche contenant le ou les agents optiques ou plusieurs couches contenant

plusieurs agents optiques différents.

On peut par exemple déposer une couche unique assurant la protection de la couche de composition photorévélable thermiquement stable vis-à-vis du rayonnement UV et de la lumière visible.

On peut aussi déposer une couche spécifique pour la protection UV et une couche supplémentaire pour une protection additionnelle dans l'UV et/ou dans le visible, cette couche supplémentaire comportant par exemple un colorant ou un agent photorévélable thermiquement instable.

On peut encore déposer une couche pour la protection UV et une couche supplémentaire comportant un composé fluorescent assurant une protection additionnelle dans l'UV.

Dans un cas particulier, on applique un film multicouche comportant une première couche de composition photorévélable thermiquement stable et une seconde couche photoprotectrice comportant un agent optique formant écran au rayonnement de révélation de la composition photorévélable thermiquement stable. Ce film peut être autoporteur ou appliqué par transfert.

La composition photorévélable thermiquement stable peut être appliquée à même les matières kératiniques ou sur une couche de base, notamment une couche de base telle que définie ci-après.

La deuxième composition peut être appliquée directement sur la couche de composition photorévélable thermiquement stable ou sur une couche intermédiaire entre les deux, comme mentionné plus haut. La deuxième composition peut elle-même être revêtue, le cas échéant, d'une couche supplémentaire.

## Choix des ingrédients des différentes couches

Dans un exemple de mise en oeuvre de l'invention, deux couches successivement appliquées, par exemple la couche de composition photorévélable thermiquement stable et la couche de composition photoprotectrice ou la couche de base et la couche de composition photorévélable thermiquement stable ou la couche de composition photorévélable thermiquement stable et la couche destinée à former un matériau protégeant le photomaquillage peuvent présenter une complémentarité physique permettant ou facilitant l'accroche de la seconde couche sur la première et/ou permettant ou facilitant l'étalement de la seconde couche sur la première.

Il peut être avantageux qu'il y ait une complémentarité d'ionicité. Ainsi, la première couche peut contenir un polymère anionique par exemple, et la seconde contient alors un composé cationique, par exemple un filtre cationique, un colorant cationique ou un composé fluorescent cationique. L'inverse est possible.

Il peut être avantageux également qu'il y ait complémentarité de tension de surface. Ainsi, la première couche peut présenter une première tension de surface, de préférence supérieure à 40 $mN.m^{-1}$, par exemple grâce à l'utilisation d'au moins un polymère hydrophile. La seconde couche peut présenter une deuxième tension de surface plus faible que la première, de préférence inférieure à 40, par exemple grâce à l'utilisation d'une composition majoritairement huileuse, siliconée ou fluorée, ou grâce à l'utilisation d'une composition aqueuse dans laquelle on a introduit un ou plusieurs tensioactifs.

On peut choisir des ingrédients (solvants, adhésifs...) pour la seconde couche qui ne sont pas solvants de la première.

On peut par exemple choisir un solvant organique (éthanol, acétone, acétate d'alkyle, huiles carbonées (isododécane par exemple), silicones volatiles pour la première couche et un solvant aqueux ou hydroalcoolique pour la seconde ou inversement.

On peut aussi choisir deux solvants organiques ou deux solvants aqueux pour les deux couches, pourvu qu'au séchage de la première couche, une transformation ait lieu. Par exemple, on emploie une première couche contenant un latex. Ce dernier va, au séchage, coalescer et rendre la première couche inerte face à l'application de la seconde couche. On peut encore employer une première couche contenant un copolymère acrylique/acrylate peu hydrosoluble et rendu hydrosoluble par une neutralisation par une base volatile telle que l'ammoniac. Après séchage de la première couche, l'ammoniac va s'évaporer et rendre la première couche résistante à l'eau.

## Couche de base

On peut appliquer sur les matières kératiniques une couche de base d'une première composition photoprotectrice contenant au moins un agent optique capable de former écran à une longueur d'onde $\lambda$ au moins temporairement, notamment une longueur d'onde appartenant à la plage à la plage 320 à 440 nm, et appliquer sur cette couche de base une deuxième composition photorévélable thermiquement stable révélable par exposition à un rayonnement au moins de longueur d'onde $\lambda$ le ou les agents optiques peuvent être choisis parmi ceux indiqués ci-dessus

La composition photoprotectrice appliquée en tant que couche de base présente par exemple, au moins au moment de son application, un pouvoir filtrant F vis-à-vis du rayonnement solaire d'au moins 2, mieux 5 ou 10.

L'utilisation de la couche de base permet de réduire le risque de tachage de la peau en rendant plus difficile la migration du ou des agents photorévélables de la composition photorévélable thermiquement stable vers les matières

kératiniques sous-jacentes.

Cette migration peut être ralentie davantage, voire empêchée, lorsque les première et deuxième compositions sont non miscibles l'une dans l'autre, l'une des compositions étant par exemple aqueuse et l'autre non aqueuse ou inversement de façon à former deux phases.

Le cas échéant, au moins une couche intermédiaire peut être appliquée sur la couche de base de façon à se situer entre la couche de composition photorévélable thermiquement stable et la couche de base.

Cette couche intermédiaire peut avoir pour effet d'améliorer la tenue de la couche de composition photorévélable thermiquement stable sur la couche de base ou au contraire d'en faciliter l'enlèvement, lors du démaquillage par exemple. La couche intermédiaire peut être une couche d'un polymère ou de cire.

La couche intermédiaire peut notamment ne pas avoir de fonction de filtration de la longueur d'onde $\lambda$ de révélation de la composition photorévélable thermiquement stable.

On peut aussi appliquer une couche d'une autre composition sous la couche de base pour faciliter son accrochage avec la peau. Ainsi, la couche de base peut ne pas être directement au contact de la peau. En variante, la couche de base est appliquée à même la peau ou autres matières kératiniques.

## Protection mécanique du photomaquillage

On peut appliquer sur les matières kératiniques au moins une couche de composition photorévélable thermiquement stable, et former grâce à une deuxième composition ou grâce à un apport d'énergie un matériau assurant une protection mécanique du photomaquillage au sein de la couche de composition photorévélable thermiquement stable.

On peut encore déposer sur la couche de composition photorévélable thermiquement stable au moins une couche de recouvrement permettant la formation d'un matériau assurant une protection mécanique du photomaquillage.

Le photomaquillage peut être réalisé avant ou après la formation du matériau assurant la protection mécanique du photomaquillage, en révélant sélectivement la couche de composition photorévélable thermiquement stable.

L'amélioration de la tenue mécanique du photomaquillage permet de retarder la dégradation de l'image formée et la perte de netteté de l'image au cours du temps est ralentie. De plus, le photomaquillage est rendu moins sensible aux frottements et aux mouvements. Le risque de transfert de la composition photorévélable thermiquement stable sur les vêtements ou d'autres régions du corps est également diminué.

On peut ainsi réaliser un photomaquillage plus durable sur des zones telles que les zones recouvertes de vêtements, par exemple le dos, le ventre, les seins, les jambes ou les fesses.

La formation dudit matériau peut se faire par une évaporation de solvant(s), par une réaction de polymérisation ou de réticulation, qui n'a pas besoin nécessairement d'être complète. Un durcissement en surface, par une polymérisation et/ou une réticulation, peut s'avérer suffisant pour améliorer la tenue.

Le matériau assurant une protection mécanique du photomaquillage est avantageusement transparent.

Lorsque le matériau recouvre la couche de composition photorévélable thermiquement stable, le matériau forme une couche d'usure qui peut, en s'usant petit à petit dans la journée, protéger le photomaquillage.

Le matériau peut également, lorsqu'il recouvre la couche de composition photorévélable thermiquement stable, contribuer à l'esthétique du photomaquillage, en apportant un effet optique supplémentaire, par exemple un effet de loupe ou de coloration.

Lorsque la composition photorévélable thermiquement stable offre la possibilité d'effacer le photomaquillage en irradiant la couche de composition photorévélable thermiquement stable à une longueur d'onde différente de celle utilisée pour révéler la composition photorévélable thermiquement stable, le matériau peut améliorer la tenue sans pour autant empêcher de faire disparaître le photomaquillage si cela est souhaité, l'utilisateur n'ayant pas à procéder à un démaquillage complet pour ce faire.

Pour former le matériau assurant la protection mécanique du photomaquillage, on peut utiliser des composés polymérisables et/ou réticulables, dans la composition photorévélable thermiquement stable et/ou dans la couche de recouvrement.

La composition photorévélable thermiquement stable peut contenir tous les composés polymérisables et/ou réticulables servant à former le matériau. Eventuellement, l'irradiation utilisée pour révéler la composition photorévélable thermiquement stable sert à la polymérisation et/ou à la réticulation.

La composition photorévélable thermiquement stable peut aussi contenir un premier agent pouvant potentiellement polymériser et/ou réticuler. Après ou avant la révélation du ou des agents photorévélables de la composition photorévélable thermiquement stable, on applique un second composé pouvant, par association avec le premier, réaliser la polymérisation ou réticulation. Eventuellement, l'irradiation sert aussi à la polymérisation et/ou réticulation.

Dans d'autres exemples de mise en oeuvre, la seconde composition est appliquée après l'application de la composition photorévélable thermiquement stable et la réalisation du photomaquillage.

L'application de la couche de recouvrement peut se faire soit avant, soit après l'irradiation servant à révéler le ou les agents photorévélables. Son épaisseur moyenne peut être d'au moins 2 $\mu$m, si possible au moins 5 $\mu$m si le

matériau est plutôt dur ou élastomérique, mieux au moins 10 μm si le matériau présente un module d'élasticité plutôt mou.

Dans le cas où les matières kératiniques sont recouvertes par une couche unique qui comprend le photomaquillage, l'épaisseur de cette couche est préférentiellement supérieure à 5 μm, et mieux 10 μm L'épaisseur est préférentiellement inférieure à 1mm.

Dans le cas où la composition photorévélable thermiquement stable intègre tout ou partie des composés pouvant potentiellement réticuler, on peut appliquer dans un second temps, avant ou après séchage de la première composition, un second composé provoquant la réticulation ou nécessaire à celle-ci. L'épaisseur de la seconde couche (exprimée après évaporation des éventuels solvants) est préférentiellement égale à au moins 20% de l'épaisseur de la première couche, et de préférence, supérieure à 50% de l'épaisseur de la première couche. L'épaisseur de la seconde couche est préférentiellement supérieure à 5 μm.

Dans le cas où la composition photorévélable thermiquement stable n'intègre aucun composé potentiellement réticulable, on peut appliquer dans un second temps une seconde composition contenant les composés produisant la réticulation. L'épaisseur de la seconde couche (exprimée après évaporation des éventuels solvants) est préférentiellement égale à au moins 10% de l'épaisseur de la première couche, et de préférence supérieure à 30% de l'épaisseur de la première couche. L'épaisseur de la seconde couche est au moins supérieure à 5 μm, et préférentiellement inférieure à 1mm.

La polymérisation et/ou réticulation permettant la formation du matériau peut être chimique ou physique.

Polymérisation et/ou réticulation chimique

On entend par réticulation chimique, le fait qu'un composé puisse soit seul, soit par réaction avec un second composé, soit par action d'un rayonnement ou d'un apport d'énergie, créer des liaisons chimiques covalentes entre les molécules. Le résultat est l'augmentation de la cohésion du matériau comportant ce composé.

Le composé peut être une molécule simple ou peut être déjà le résultat de la combinaison de plusieurs molécules, par exemple des oligomères ou polymères. Le composé peut porter une ou plusieurs fonctions réactives.

On privilégie les molécules donnant après réticulation un matériau solide et/ou déformable mais élastomérique.

Les fonctions chimiques peuvent réagir sur une autre fonction de même nature ou réagir avec une autre fonction chimique.

Réaction sur une autre fonction de même nature

Il s'agit par exemple des fonctions éthyléniques, notamment les fonctions acrylates, acryliques, methacrylates, méthacryliques ou styrène.

Ces molécules demandent en général une forme d'activation extérieure pour réagir, par exemple de la lumière, de la chaleur, l'application d'un catalyseur, ou une combinaison avec des photoinitiateurs et éventuellement des photosensibilisateurs destinés à élargir la gamme d'action des photoinitiateurs. Des compositions photopolymérisables et/ou photoréticulables sont décrites par exemple dans les brevets CA 1 306 954 et US 5 456 905.

On peut utiliser les composés polymériques portant des fonctions réactives éthyléniques décrits dans le brevet EP 1 247 515.

Les fonctions éthyléniques peuvent être activées par un groupe attracteur de façon à accélérer les réactions et rendre inutile l'apport d'une activation extérieure. C'est typiquement le cas du monomère éthylcyanoacrylate, pour lequel la seule présence d'un catalyseur comme de l'eau permet la réaction.

Les fonctions éthyléniques peuvent être activées modérément, par exemple par un groupe attracteur. L'avantage est que la réaction nécessite une activation extérieure, ce qui est intéressant pour contrôler le démarrage et le rendement de la réaction, mais ne nécessite pas de photoamorceur. Par exemple, il s'agit de monomères cyanoacrylates, et en particulier des monomères cyanoacrylates dont le groupe porté par la fonction ester contient au moins 2, si possible 4, enchainements carbonés.

Sont appréciées les molécules demandant une activation extérieure telle que la lumière, mais ne demandant pas de photoinitiateur. Ainsi, sont spécialement préférées les molécules capables de réagir par photodimérisation, telles que celles décrites dans le brevet EP 1 572 139, en particulier celles portant des fonctions telles que

1) stylbazoliums.

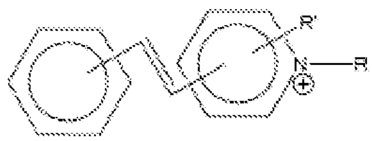

où

- R représente l'atome d'hydrogène, un groupement alkyle ou hydroxyalkyle, et
- R' représente l'atome d'hydrogène ou un groupement alkyl,

2) styrylazoliums :

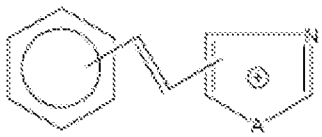

où
A désigne un atome de soufre, un atome d'oxygène, ou un groupement NR' ou C(R')2, R et R' étant tels que définis précédemment,
3) chalcone ,
4) (thio)cinnamate et (thio)cinnamamide,
5) maléimide,
6) (thio)coumarine,
7) thymine,
8) uracile,
9) butadiène
10) anthracène,
11) pyridone,
12) pyrrolizinone,
13) sels d'acridizinium,
14) furanone,
15) phénylbenzoxazole,
16) styrylpyrazine.

Les réactions s'effectuant sur une autre fonction de même nature ne sont pas limitées aux réactions impliquant des fonctions éthyléniques.
Sont aussi appréciés les composés pouvant réagir par condensation, tels que

- des groupes siloxanes, et en particulier les fonctions dialkoxy ou dihydroxy silanes, les fonctions trialkoxy ou trihydroxysilanes. On peut utiliser des molécules porteuses de fonctions alkyltrialkoxysilane ou dialkyltrialkoxysilane, et en particulier des fonctions alkylalkoxysilane où le groupe alkyl porte une fonction hydrosolubilisante comme une amine, par exemple une molécule comme l'aminotriéthoxysilane ou aminotriéthoxysilane ou des molécules porteuses de telles fonctions. En plus des petites molécules à base de siloxanes (monomères ou oligomères) on peut utiliser des composés de masse plus importante, en particulier ceux décrits dans le brevet FR 2 910 315.
- des sol-gels à base de titane.

Avec ces molécules, on peut contrôler le démarrage et le rendement de la réaction.
Sont aussi appréciés les composés pouvant réagir par oxydation, tels que les composés aromatiques portant au moins deux fonctions hydroxyl, ou une fonction hydroxyl et une fonction amine, ou une fonction hydroxyl, par exemple le cathécol ou dihydroxyindole. L'agent oxydant peut être l'oxygène de l'air ou un autre oxydant tel que de l'eau oxygénée par exemple.

Réaction sur une autre fonction

Les molécules qui réagissent dans ce cas présentent deux types de fonctions, complémentaires. Il peut s'agir de systèmes où l'on met en contact des molécules portant des fonctions FA et des molécules portant des fonctions FB pouvant réagir avec les fonctions FA.

Il peut aussi s'agir de molécules portant sur la même structure une ou plusieurs fonctions FA et une ou plusieurs fonctions FB.

La fonction FA peut être choisie par exemple parmi :

- epoxyde,
- aziridine,
- vinyle et vinyle activée, en particulier, acrylonitrile, esters acrylique et métacrylique,
- acide et esters crotonique, acide et esters cinnamique, styrène et dérivés, butadiène,
- éthers de vinyle, vinylcétone, esters maléiques, vinyl sulfones, maléimides,
- anhydride, chlorure d'acide et esters d'acide carboxylique,
- aldehydes,
- acetals, hémi-acétals,
- aminals, hémi aminals,
- cetones, alpha-hydroxycétones, alpha-halocétones,
- lactones, thiolactones,
- isocyanate,
- thiocyanate,
- imines,
- imides, en particulier, succinimide, glutimide,
- N hydroxysuccinimide esters,
- imidates,
- thiosulfate,
- oxazine et oxazoline,
- oxazinium et oxazolinium,
- halogenures d'alkyle en C1 à C30 ou d'aryle ou aralkyle en C6 à C30 de formule RX, avec X = I, Br, Cl,
- halogenure de cycle insaturé, carboné ou hétérocycle, notamment les chlorotriazine,
- chloropyrimidine, chloroquinoxaline, chlorobenzotriazole,
- halogenure de sulfonyle : RSO2Cl ou F, R étant un alkyle en C1 à C30.

A titre d'illustration, on peut citer les molécules porteuses de fonctions du groupe FA :

- copolymere méthyl-vinyl éther/ anhydride maléique, en particulier commercialisé par exemple par la société ISP sous le nom de Gantrez,
- polymethacrylate de glycidyle, en particulier commercialisé par Polysciences,
- polydimethylsiloxane de glycidyle, en particulier commercialisé par la société Shinetsu (référence X-2Z-173 FX ou DX),
- polyamidoamine époxy, par exemple commercialisé par la société Hercules sous le nom de Delsette 101, le Kymène 450 de chez Hercules,
- epoxy-dextrane,
- polysaccharides polyaldéhydes obtenus par oxydation de polysaccharides par NaIO4 (Bioconjugate Techniques ; Hermanson GT, Academic Press, 1996),

La fonction FB peut être choisie parmi les fonctions XHn avec X= O, N, S, COO et n= 1 ou 2, notamment les alcool, amine, thiol et acide carboxylique.

A titre d'exemple, on peut citer comme molécules porteuses de fonctions de type FB :

- dendrimere PAMAM , en particulier commercialisé par la société Dendritech, DSM, Sigma- Aldrich (STARBURST, PAMAM DENDRIMER, G(2, O) de la société DENDRITECH),
- dendrimere à fonctions hydroxy, en particulier commercialisé par la société Perstorp, DSM, (exemple : HBP TMP core 2 Generation PERSTORP)
- PEI (polyéthylène-imine), en particulier commercialisé par BASF, sous le nom de Lupasol,
- PEI-Thiol,
- polylysine, en particulier commercialisé par la société Chisso

- HP cellulose, tel que KLUCELEF de la société AQUALON),
- amino-dextrane, par exemple commercialisé par la société Carbomer,
- amino-cellulose, par exemple ceux décrits dans WO01/25283 de la société BASF,
- PVA (polyvinylacétal), par exemple AIRVOL 540 de la société AIRPRODUCTS CHEMICAL)
- amino PVA, par exemple commercialisé par la société Carbomer ,
- chitosane,

Sont compris aussi dans ce deuxième cas, les molécules pouvant réagir par réaction d'hydrosilylation :

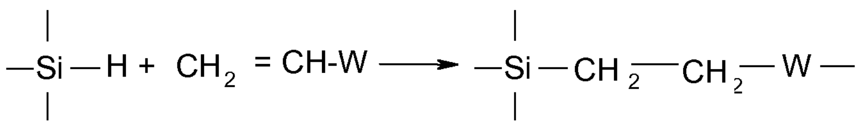

(W représente une chaine carbonée ou siliconée par exemple).

Les précisions sur les deux ingrédients, les molécules commerciales accessibles, les conditions de catalyseurs et les conditions d'utilisation sont décrites dans la demande de brevet FR 2 910 315.

Dans un cas particulier, on utilise une molécule déjà présente sur la peau, ou excrétée par la peau, comme réactif ou agent catalyseur. Typiquement, l'eau, qui peut aider à la réaction des cyanoacrylates par exemple, ou de certaines réactions impliquant des siloxanes.

Dans un autre cas particulier, on utilise une molécule présente dans l'air ambiant, comme réactif ou agent catalyseur. Typiquement, l'oxygène impliqué dans la réaction de réticulation des certaines huiles comme les huiles siccatives, et en particulier des huiles végétales siccatives comme l'huile de lin, l'huile de Bois de Chine (ou Canton), l'huile d'oïticica, l'huile de vernonia, l'huile d'oeillette, l'huile de grenade, l'huile de calendula, ou les résines alkydes. Les réactions peuvent être accélérées par l'emploi de catalyseurs, tels que des sels de cobalt, de manganese, de calcium, de zirconium, de zinc, de strontium, de plomb, de lithium, de fer, de cerium, de barium, d'étain, sous la forme par exemple d'octoate, de linoleate, ou d'octanoate.

Dans un autre cas particulier, on utilise des molécules qui par réarrangement, vont se lier les unes aux autres. Ainsi, on peut utiliser des molécules portant un disulfure interne. Par ouverture du disulfure interne et réaction de ces disulfures, on peut créer de nouvelles liaisons covalentes entre les molécules.

On peut utiliser des catalyseurs pour accélérer les réactions. Par exemple, des sels de métaux comme le manganèse, le cuivre, le fer, le platine, les titanates, ou des enzymes telles que des oxydases ou des laccases.

Dans le cas de fonctions chimiques réagissant sur une autre fonction de même nature ou non, plusieurs modes d'application sont possibles.

Par exemple, tous les ingrédients réagissant sont intégrés dans la composition photorévélable thermiquement stable, ou tous les ingrédients sont intégrés dans la composition photorévélable thermiquement stable à l'exception d'un ou plusieurs composés, par exemple soit l'un des composés, soit un catalyseur. Aucun des ingrédients peut n'être intégré dans la composition de photorévélable thermiquement stable, étant tous appliqués en un ou plusieurs temps, après application de la composition photorévélable thermiquement stable, et préférentiellement après réalisation du photomaquillage.

Réticulation physique

La réticulation peut être physique, grâce à des ingrédients capables de créer des liaisons physiques solides entre les molécules et conférant au matériau final une résistance à l'eau. Ces liaisons, non covalentes, sont de type ioniques ou hydrogènes.

A titre d'exemple, on peut citer les mélanges avec un sel type di ou polyvalent, par exemple de calcium, zinc, strontium ou aluminium.

Par exemple, on peut mélanger un composé A comme un dérivé d'alginate et un composé B comme un sel de calcium. Le dérivé d'alginate est par exemple contenu dans la composition photorévélable thermiquement stable. On applique dans un second temps, sous forme de spray par exemple, une solution aqueuse de chlorure de calcium pour provoquer la réticulation.

On peut encore citer les molécules capables de créer des liaisons hydrogène fortes, comme par exemple les copolymères blocs polysiloxanes/polyurées, et notamment ceux de formules :

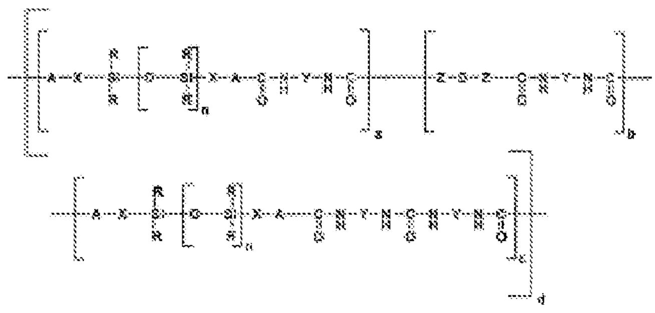

Où:

- R représente un radical hydrocarboné monovalent de 1 à 20 atomes de carbone, pouvant être substitué par un ou plusieurs atomes de fluor ou de chlore,
- X représente un radical alkylène ayant 1 à 20 atomes de carbone, dans lequel des unités méthylène non voisines peuvent être remplacées par des radicaux -O-,
- A représente un atome d'oxygène ou un radical amino-NR'-,
- Z représente un atome d'oxygène ou un radical amino -NR'-,
- R' représente hydrogène ou un radical alkyle ayant 1 à 10 atomes de carbone,
- Y représente un radical hydrocarboné bivalent, le cas échéant substitué par le fluor ou le chlore, ayant 1 à 20 atomes de carbone,
- D représente un radical alkylène, le cas échéant substitué par fluor, chlore, alkyle en C1-C6 ou ester alkylique en C1-C6, ayant 1 à 700 atomes de carbone, dans lequel des unités méthylène non voisines peuvent être remplacées par des radicaux -O-, -COO-, - OCO- ou -OCOO-,
- n est un nombre allant de 1 à 4000,
- a est un nombre d'au moins 1,
- b est un nombre allant de 0 à 40,
- c est un nombre allant de 0 à 30, et
- d est un nombre supérieur à 0.

Des précisions sur les fonctions, les molécules commercialement accessibles et les conditions de mise en oeuvre sont données dans le brevet EP 759 812.

<u>Composés de réticulation amenant à la formation d'un revêtement spécialement résistant</u>

Que ce soit par réticulation chimique ou physique, on peut choisir les composés de réticulation pour qu'ils assurent la meilleure résistance possible, notamment à l'eau et à l'humidité.

Ainsi, on peut réaliser des revêtements très hydrophobes et ce, notamment pour traiter les parties du corps qui transpirent le plus, comme le buste ou les aisselles par exemple.

Par exemple, on emploie un premier ingrédient réactif FA de type polyols, comme par exemple un dérivé de cellulose, et un second ingrédient réactif FB, de type perfluoroalkyltriéthoxysilane. Dans ce cas précis, on réalise l'application en deux temps. Le polyol est introduit dans la composition photorévélable thermiquement stable. On applique sur la composition photorévélable thermiquement stable une composition de recouvrement contenant l'ingrédient FB.

Par exemple encore, on emploie un système capable de donner un revêtement réticulé, et contenant aussi des particules hydrophobes. Une illustration de ces associations est l'association de particules hydrophobes avec des technologies de condensation ou d'hydrosilylation telles que décrites dans le brevet FR 2 910 315. Les particules solides utilisables peuvent être d'origine minérale ou organique, poreuses ou non poreuses, colorées ou non colorées. Elles peuvent être de morphologie quelconque, de préférence sphérique. Les particules peuvent être naturellement hydrophobes, ce qui est le cas de la poudre de PTFE par exemple ou peuvent être rendues hydrophobes par des enrobages, notamment hydrocarbonés, siliconés, fluorés ou encore fluorosiliconés.

On peut aussi réaliser des revêtements amenant une meilleure résistance au sébum et aux corps gras, à base d'oxyde ou de sels de zinc par exemple, ou des revêtements rendus plus résistants à l'élongation ou au déchirement. Ces améliorations peuvent être utiles pour des applications sur les parties du corps les plus sujettes aux mouvements, telles que les lèvres, les mains, les aisselles, le cou ou toutes zones proches des articulations.

La résistance à l'élongation peut être acquise par l'emploi d'ingrédients de réticulation donnant par exemple un matériau à caractère élastomérique. On peut aussi intégrer dans la ou les compositions des composés non réactifs, amenant un caractère élastomérique, par exemple un polymère élastomère comme par exemple un latex naturel déprotéiné, ou des fibres.

Un cas particulier est d'imprégner avec les ingrédients de réticulation un tissu tissé ou non tissé. On peut apposer sur la peau, avant pendant ou après l'application de la composition de photomaquillage, un tissu tissé ou non tissé. L'imprégnation de la composition dans le tissu apporte une résistance mécanique.

On peut aussi réaliser une association de tissu et de composition de photomaquillage, puis, une fois réalisée, l'appliquer sur la peau, avec ou sans l'aide d'un adhésif.

On peut intégrer dans les compositions des actifs lubrifiants et notamment des actifs lubrifiants solides tels que le nitrure de bore ou d'aluminium par exemple.

On peut aussi intégrer des charges solides, et notamment des charges hydrophiles ou rendues hydrophiles, telles que des particules d'oxyde de métaux, d'hydroxydes de métaux, de carbonates de métaux ou des particules organiques. Ces charges peuvent conférer une résistance additionnelle à l'abrasion.

**<u>Couche de recouvrement formant couche d'usure</u>**

La couche de revêtement peut former le matériau de protection mécanique au-dessus de la couche de composition photorévélable thermiquement stable et agir comme une couche d'usure.

Dans ce cas, la couche de revêtement est avantageusement cohésive après évaporation des éventuels solvants et peut être appliquée avant ou après l'irradiation.

On entend par cohésive, le fait que la couche résiste aux contacts. Par exemple, si l'on approche une sonde plate de 1 $cm^2$ de surface de la couche de revêtement, de façon qu'elle entre en contact avec une pression de 10 $N/cm^2$, puis que l'on retire après un temps de contact de 5 secondes la sonde, celle-ci ne doit pas entraîner de matière. Ainsi, sont exclus les composés huileux.

La couche de revêtement n'est pas collante une fois les solvants évaporés. On entend par non collante le fait que la couche n'offre pas de résistance au retrait. Par exemple, si l'on approche de la couche une sonde plate de surface de 1 $cm^2$, de façon qu'elle entre en contact avec une pression de 10 $N/cm^2$, puis qu'on la retire après un temps de contact de 5 secondes, celle-ci ne doit pas demander de force de résistance au moment du retrait. Ainsi, sont exclus les composés dits PSA (*pressure sensitive adhesive*).

Le matériau formant la couche de revêtement peut présenter un module d'élasticité inférieur à 500MPa et supérieur à 100kPa, préférentiellement entre 200 MPa et 1MPa.

Son épaisseur moyenne est d'au moins 1 $\mu$m, si possible au moins 2 $\mu$m si le matériau présente un module d'élasticité supérieur à 10MPa. Son épaisseur moyenne est d'au moins 2 $\mu$m, si possible au moins 5 $\mu$m si le matériau présente un module d'élasticité inférieur à 10 MPa.

Dans le cas où le matériau formant la couche de revêtement est élastomérique, c'est-à-dire présentant une déformation maximale d'au moins 400% avant rupture et présentant une recouvrance élastique d'au moins 90% après un temps d'attente de 1 minute, l'épaisseur moyenne est préférentiellement d'au moins 1 $\mu$m, même si le module d'élasticité est inférieur à 10 MPa.

On entend par recouvrance élastique, le niveau de retour à sa longueur initiale, d'une éprouvette après une traction de 40% puis relachement de la contrainte. Ainsi, si la longueur initiale de l'éprouvette est L0, et la longueur après traction de 40% et relachement de la contrainte est L(t), la recouvrance R(t) à l'instant t, compté après relachement, est égale à : 100* (1-( L(t)-L0) /L0 )/0,4).

Ainsi, si L(t) = L0, alors R(t)=100. Si L(t)=1,4*L0, alors R(t)=0

Le test de recouvrance est réalisé en préparant d'abord une éprouvette d'environ 200$\mu$m d'épaisseur, de 6 cm de long, et 1 cm de large. Si besoin, l'éprouvette est éventuellement réalisée sur un film de support dont l'impact mécanique est jugé faible au regard des propriétés mécaniques de l'éprouvette.

L'éprouvette est soumise à une traction de 40% de sa longueur à la vitesse de 0,1 mm/s. Puis la contrainte est relâchée et on attend 1 minute.

Préférentiellement, la couche de revêtement est appliquée avec un solvant majoritaire différent de celui utilisé pour la couche de composition photorévélable thermiquement stable. Toutefois, cette condition peut être contournée notamment dans le cas de l'utilisation, pour la couche de composition photorévélable thermiquement stable, de composés réticulants ou coalesçants.

Par exemple, si la couche de composition photorévélable thermiquement stable contient un latex de Tg < 40°C et de l'eau par exemple, la couche de revêtement peut être aussi à base d'eau.

Si la couche de composition photorévélable thermiquement stable contient un solvant et un composé capable

de réticuler, comme par exemple ceux décrits précédemment, la couche de revêtement peut contenir le même solvant.

Pour aider à la bonne réalisation de la couche de revêtement, on peut apporter avant son application une irradiation lumineuse ou la chaleur par exemple. On peut aussi déposer une couche intermédiaire faite par exemple d'une résine ou tout autre produit aidant à l'adhésion, comme par exemple un adhésif ou certaines poudres, notamment celles qui par leur granulométrie, aident la couche supérieure à s'agripper.

On peut apporter après l'application de la couche de revêtement, une irradiation lumineuse ou de la chaleur.

La couche de revêtement peut être éliminée progressivement. Ainsi, la couche de photomaquillage n'est pas altérée dans le temps et permet au photomaquillage de garder toute sa précision.

Ingrédients de la couche de revêtement formant couche d'usure

Les composés pouvant entrer dans la réalisation d'une telle couche de revêtement sont des polymères, par exemple des poly(méth-)acryliques, poly(méth-)acrylates, des polyuréthannes, des polyesters, des polystyrènes ou des copolymères sous forme soluble ou dispersée, par exemple choisis parmi le Mexomer, l'ultrahold Strong DR 25, le 28-29-30, la Gantrez, l'Amerhold DR 25, l'amphomer, le Luviset Si Pur, l'AQ 38 ou AQ 48.

Les polymères peuvent porter des groupes latéraux ou terminaux pour ajuster leur dureté. Par exemple, le matériau formant la couche de recouvrement peut comporter des polymères acrylates à fonctions siliconées, comme le VS 80 par exemple.

Les polymères peuvent être des polymères naturels ou des polymères naturels modifiés, par exemple des polymères polyosiques, comme des gommes de guar, des gommes de caroube, des dérivés de cellulose, comme le HPMCP ou des protéines.

Les polymères peuvent être des polymères hydrocarbonés.

Les polymères peuvent être des silicones, comme des gommes de silicone par exemple.

Comme la plupart des polymères n'ont pas toujours les qualités intrinsèques pour donner la durée requise, il peut être utile d'adjoindre un plastifiant.

En plus des plastifiants habituellement utilisés, par exemple éther de glycol (Monométhyl ether de tropopylène glycol (nommé PPG3 méthyl ether par la société DOW CHEMICAL) ou glycérine, on peut inclure certains solvants non volatils, comme le carbonate de propylène, des alcools, des huiles siliconées ou carbonées.

Les quantités de plastifiants sont calculées en fonction du polymère et de ses qualités intrinsèques. Typiquement (% par rapport au poids de polymère) :

| | Ether glycol | Glycérine |
|---|---|---|
| Ultrahold Strong DR 25 | 5% | 10% |
| Mexomer | 4% | 8% |
| AQ 48 | 1% | 2% |
| Luviset Si Pur | 3% | 5% |
| VS 80 | 5% | 10% |

On peut inclure dans la composition des particules, minérales ou organiques, pouvant prolonger la durée de vie de la couche d'usure sans toutefois lui faire perdre ses qualités. Les particules ne provoquent pas de tiraillements de la peau, mais peuvent toutefois provoquer des phénomènes d'écaillement ou l'apparition de boulettes. Ainsi, préférentiellement, on ne dépasse pas une concentration massique de 40% en particules (particules pouvant coalescer non comprises).

On peut inclure des agents de rhéologie aidant à l'application.

On peut aussi inclure des agents d'étalement comme des tensioactifs ou certains solvants de température d'ébullition comprise entre 80 et 200°C. Ces solvants présentent l'avantage de ralentir la prise en masse de la composition tout en s'éliminant dans le temps.

Concentrations et épaisseurs après séchage

Les concentrations des différents ingrédients peuvent être ajustées de façon que les épaisseurs après séchage soient, compte tenu des quantités appliquées, en accord avec les spécifications données plus haut.

Par exemple, en supposant que l'on applique 20 $mg/cm^2$ de composition fluide à étaler, que la composition contienne 10% de matière sèche, on peut déposer environ 2 $mg/cm^2$. Si la densité est d'environ 1, cela correspondant à une épaisseur d'environ 20 $\mu$m.

Dans un autre exemple, si l'on suppose que l'on pulvérise à 30 cm du visage pendant 4s une composition aérosol renfermant 20% de matière sèche, on va déposer environ 0,4g pour 400 $cm^2$, soit 1 mg par $cm^2$. Si la densité est d'environ 1, l'épaisseur de la couche ainsi déposée sera de 10$\mu$m environ.
Ainsi, selon les modes d'application et les formes galéniques, les concentrations en matière sèche peuvent aller de 1 à 50%.
La composition de revêtement peut être sèche.

**Autres ingrédients**

En plus des ingrédients déjà mentionnés, chaque composition peut contenir des ingrédients permettant ou aidant l'étalement sur les matières kératiniques, plus particulièrement la peau, amenant un soin, un confort, par exemple une odeur ou une douceur, aidant à l'élimination au moment du lavage, par exemple un ou plusieurs tensioactifs, limitant la pénétration des ingrédients dans la peau, par exemple des astringents, ou amenant d'autres fonctionnalités cosmétiques, par exemple de l'hydratation, de la couleur, de la brillance et /ou limitant les impacts de la filtration UV, par exemple un autobronzant ou un activateur de la vitamine D.

**Démaquillage**

Au cours du démaquillage, l'utilisateur peut laisser des traces de composition photorévélable thermiquement stable non révélée. Or, ces traces peuvent être amenées à se révéler par la suite, par exemple après quelques heures passées à la lumière ambiante. A ce moment, il peut être difficile pour l'utilisateur de procéder à un complément de démaquillage.
Pour remédier à ce problème, il peut être avantageux d'appliquer pendant ou après le démaquillage, au moins un agent optique formant écran à au moins une longueur d'onde $\lambda$ servant à révéler la composition photorévélable thermiquement stable.
On peut réappliquer un tel agent optique plusieurs fois de suite, le cas échéant. L'agent optique peut appartenir à une composition démaquillante.
Par « former écran à la longueur d'onde $\lambda$ », il faut comprendre que l'agent optique atténue d'au moins un facteur 2 le rayonnement de longueur d'onde $\lambda$, la mesure étant effectuée par le biais d'un appareil apte à mesurer le spectre d'absorption en restreignant la lumière d'irradiation à une zone de longueur d'onde centrée autour de $\lambda$, comme détaillé plus haut. L'application pendant et mieux, après, le démaquillage de l'agent optique empêche les traces d'agent photorévélable de se révéler et diminue le risque de tacher les matières kératiniques ou les vêtements.
Les matières kératiniques peuvent ne pas être lavées dans l'heure suivant l'application de l'agent optique. Quand plus tard l'utilisateur procède à un lavage, les traces d'agent photorévélable non révélé et protégées par l'agent optique pourront être éliminées.
Un autre avantage lié à l'application de l'agent optique est que l'on évite, au moment où l'on réalise un nouveau photomaquillage, que certaines parties non révélées du précédent photomaquillage et encore présentes se révèlent lors de l'exposition au rayonnement utilisé pour réaliser le nouveau photomaquillage.
L'agent optique peut être appliqué après le démaquillage. L'agent optique peut aussi entrer dans la formulation d'une composition démaquillante utilisée pour le démaquillage.
La longueur d'onde $\lambda$ peut appartenir au spectre UV ou proche UV (290 nm à 400 nm), notamment à l'intervalle allant de 320 nm à 440 nm.
La composition démaquillante peut être un produit de démaquillage classique à base de tensioactifs, ou un produit de démaquillage particulier adapté aux composés de la composition photorévélable thermiquement stable, et peut comporter un solvant, par exemple l'acétate d'éthyle ou de butyle, l'acétone, l'éthanol et leurs mélanges, et plus généralement tout solvant choisi parmi les solvants organiques cosmétiquement acceptables (tolérance, toxicologie et toucher acceptables). Ces solvants organiques peuvent représenter de 0 % à 98 % du poids total de la composition. Ils peuvent être choisis dans le groupe constitué par les solvants organiques hydrophiles, les solvants organiques lipophiles, les solvants amphiphiles ou leurs mélanges. Parmi les solvants organiques hydrophiles, on peut citer par exemple des monoalcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone ; les éthers de glycol comme le diéthylène glycol mono-méthyl ou mono-éthyl éther et les éthers de propylène glycol comme le dipropylène glycol méthyl éther.
Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate. Comme solvants organiques lipophiles, on peut citer par exemple les esters gras tels que l'adipate de diisopropyle, l'adipate de dioctyle, les benzoates d'alkyle, le myristate d'isopropyle, le palmitate d'isopropyle, le

stéarate de butyle, le laurate d'hexyle, l'isononanoate d'isononyle, le palmitate de 2-éthyl hexyle, le laurate de 2-hexyl-décyle, le palmitate de 2-octyl-décyle, le myristate de 2-octyl-dodécyle, le succinate de di-(éthyl-2-hexyle), le malate de diisostéaryle, le lactate de 2-octyl-dodécyle, le triisostéarate de glycérine, le triisostéarate de diglycérine.

La composition démaquillante peut encore comporter :

- une huile, par exemple sous forme de microémulsion,
- un agent de pH si le ou les composés utilisés pour maintenir le ou les agents photorévélables sur la peau sont sensibles au pH, ce qui est le cas du carbopol par exemple, ou
- un liquide ionique.

Parmi les tensioactifs anioniques qui peuvent être utilisés seuls ou en mélange dans la composition démaquillante, on peut citer en particulier les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, $\alpha$-oléfines sulfonates, paraffines sulfonates, alcoylsulfosuccinates, alcoyléthersulfosuccinates, alcoylamides sulfosuccinates, alcoylsulfosuccinamates, alcoylsulfoacétates, alcoylpolyglycérol carboxylates, alcoylphosphates/alcoylétherphosphates, acylsarcosinates, alcoylpolypeptidates, alcoylamidopolypeptidates, acyliséthionates, alcoyllaurates.

Le radical alcoyle ou acyle dans tous ces composés désigne généralement une chaîne de 12 à 18 atomes de carbone.

On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques répondant à la formule : $Alk-(OCH_2-CH_2)_n-OCH_2-COOH$ sous forme acide ou salifiée où le substituant Alk correspond à une chaîne linéaire ayant de 12 à 18 atomes de carbone et où n est un nombre entier compris entre 5 et 15.

Parmi les tensioactifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose; les alkylpolyglycosides et les alkylamides des sucres aminés; les produits de condensation d'un $\alpha$-diol, d'un monoalcool, d'un alcoylphénol, d'un amide ou d'un diglycolamide avec le glycidol ou un précurseur de glycidol.

La composition de démaquillage qui contient l'agent optique peut être formulée de façon à permettre à l'agent optique de se déposer au moment du rinçage, par exemple par effet de coacervation, cet effet pouvant être obtenu par exemple en utilisant des tensioactifs et polymères d'ionicité complémentaires, par exemple PC/PA, TC/TA, TC/PA, TA/PC, avec éventuellement des tensioactifs amphotères et non ioniques pour faciliter le dépôt. Les PC sont typiquement des composés tels que les gommes de guar cationiques (Jaguar C13S par exemple) ou des composés artificiels, comme le JR 400 ou le ionène. Les TC sont typiquement des composés chaines quaternaires (et en particulier des groupes triméthylammonium) et chaine grasse (C6 à C30). Les PA peuvent être des polymères multianioniques tels que des polymères ou copolymères acrylates ou méthacrylates ou des polymères à groupes sulfoniques. Les TA sont des tensioactifs anioniques tels que les tensioactifs carboxyliques ou sulfate ou sulfonique (LES, LS).

La composition démaquillante peut être appliquée à l'aide de tout support adapté, notamment capable de l'absorber, par exemple un disque démaquillant fibreux, par exemple un tissé ou non tissé, un feutre, de l'ouate, un film floqué, une éponge, une lingette, le support utilisé pour le démaquillage est avantageusement éliminé après l'opération de maquillage.

La composition démaquillante peut être contenue dans un récipient et prélevée au fur et à mesure à chaque démaquillage. En variante, la composition démaquillante imprègne le support utilisé pour le démaquillage, le support pouvant être dans ce cas conditionné par exemple dans un emballage étanche. Après l'utilisation de la composition démaquillante, les matières kératiniques peuvent ne pas être rincées. En variante, elles peuvent l'être. Le rinçage peut s'effectuer par exemple à l'eau courante, sans adjonction d'un savon.

**Exemples proposés**

Les proportions indiquées sont massiques.

Formule 1 (composition photochromique) :

| | | |
|---|---|---|
| Diaryléthene * (1,2-BIS(2,4-DIMETHYL-5-PHENYL-3-THIENYL)-3,3,4,4,5,5-HEXAFLUOROCYCLOPETENE) | | 0,8% |
| Cellulose | | 4% |
| Acétone | qs | 100% |

* :

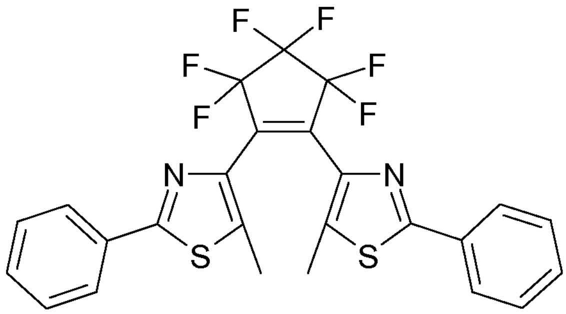

Formule 2 (composition photoprotectrice destinée à former la couche de base) :

| | | |
|---|---|---|
| Parsol 1789 (filtre solaire non photostable) | | 5% |
| Acétate d'éthyle | qs | 100% |

On introduit ce mélange filtre et solvant dans un aérosol, pressurisé avec du DME (65/35).

Négatif

On peut créer un négatif pour le photomaquillage de la façon suivante. On réalise un fichier PowerPoint® représentant un damier où chaque carré fait environ 3 mm de côté. Puis on imprime ce fichier sur un transparent avec une imprimante laser. Le transparent lorsqu'il n'est pas imprimé, laisse passer les UV dans la bande d'activation UVA, autour de 365 nm.

Irradiateur

L'irradiateur UV utilisé est par exemple une lampe de Wood, vendue par la société Bioblock Scientific, de référence VL 6L qui délivre à peu près 6W sur environ 75 $cm^2$, aux alentours de 365 nm. La puissance reçue, mesurée avec un wattmètre, est de 2,25 mW par $cm^2$ à 3 cm de l'appareil.

Essais

Les essais sont réalisés à la lumière ambiante dans une salle fermée.

On utilise comme support de photomaquillages des têtes en plastique destinées aux essais de coiffure, par exemple des têtes malléables de marque Suzy, disponibles auprès de la société Cosmey International.

On applique sur ces têtes les compositions que l'on laisse sécher une minute.

On plaque le négatif sur la surface en le maintenant à la main, puis on approche l'irradiateur à environ 3 cm

de la surface. On irradie pendant t secondes, correspondant à une énergie reçue d'environ 2,25 t mJ/cm$^2$.

On évalue la qualité du photomaquillage à l'oeil en prenant soin de regarder la qualité du motif en damier.

Après démaquillage à l'eau savonneuse, on observe la couleur résiduelle en plaçant d'abord la tête en plastique dans une pièce éclairée. On place ensuite la tête en plastique pendant environ 1 heure à la lumière extérieure.

Essai 1 :

On réalise un premier photomaquillage en appliquant la formule 1 directement sur la peau le support de la tête en plastique, en laissant sécher 1 minute, puis en illuminant la zone avec l'irradiateur UV avec, sur la peau, le négatif représentant le motif à reproduire. L'irradiation prend environ 10 secondes et délivre 22,5 mJ/cm$^2$.

Essai 2 :

On réalise un second photomaquillage identique au précédent, à la différence que l'on pulvérise la formule 2 dix minutes avant application de la composition photochromique.

On laisse les deux photomaquillages en ambiance éclairage intérieur de maison, pendant 24 heures, puis on les démaquille avec une composition aqueuse de tensioactif non ionique et on laisse 1 heure à la lumière extérieure. On s'aperçoit que certaines zones se colorent un peu dans le cas de l'essai 1. A l'inverse, aucune zone ne se colore dans le cas de l'essai 2.

L'invention n'est pas limitée aux exemples décrits.

Par exemple, la première composition servant à former la couche de base peut éventuellement être utilisée une deuxième fois en étant appliquée sur la couche de composition photorévélable thermiquement stable, en tant que couche photoprotectrice. Cette application peut avoir lieu avant l'irradiation servant à réaliser le photomaquillage, ou après. Lorsque l'application a lieu avant, l'exposition est plus longue et/ou réalisée avec une fluence plus grande pour compenser l'absorption résultant de la présence de cette couche sur la couche de composition photorévélable thermiquement stable.

D'autres zones du corps que le visage peuvent être traitées. Tous les exemples se référant à un traitement du visage valent également pour traiter d'autres régions.

L'expression « comportant un » doit se comprendre comme étant synonyme de « comportant au moins un ».

**Revendications**

1. Procédé de photomaquillage des matières kératiniques humaines, dans lequel :

   a) on applique sur les matières kératiniques une couche de base d'une première composition contenant au moins un agent optique capable de former écran à une longueur d'onde λ au moins temporairement,
   b) on applique sur la couche de base une deuxième composition photorévélable thermiquement stable révélable par exposition à un rayonnement au moins de longueur d'onde λ.

2. Procédé selon la revendication 1, l'agent optique étant un filtre solaire non photostable, d'indice de photostabilité inférieur ou égal à 80 %.

3. Procédé selon la revendication 2, le filtre solaire ayant initialement un pouvoir filtrant F vis-à-vis du rayonnement UV solaire supérieur ou égal à 2, mieux 5 ou 10.

4. Procédé selon l'une quelconque des revendications précédentes, la couche de base étant appliquée sur une surface plus étendue que la composition photorévélable thermiquement stable.

5. Procédé selon l'une quelconque des revendications 1 à 4, la couche de base étant appliquée à même la peau.

6. Procédé selon l'une quelconque des revendications 1 à 4, la composition photorévélable thermiquement stable comportant un agent photorévélable thermiquement stable.

7. Procédé selon la revendication 6, l'agent photorévélable étant choisi parmi les diaryléthènes et les fulgides.

8. Procédé selon l'une quelconque des revendications précédentes, la longueur d'onde λ appartenant au spectre UV ou proche UV.

**9.** Procédé selon l'une quelconque des revendications 1 à 8, la couche de base étant non aqueuse et la composition photorévélable thermiquement stable étant aqueuse, ou inversement.

**10.** Procédé selon l'une quelconque des revendications 1 à 9, la composition photorévélable thermiquement stable étant révélée à l'aide d'un imageur matriciel adressable.

**11.** Ensemble comportant, au sein d'un même conditionnement :

- une première composition à appliquer sur les matières kératiniques, pour former une couche de base, contenant au moins un agent optique capable de former écran à une longueur d'onde λ appartenant à l'intervalle allant de 280 nm à 440 nm, mieux de 320 nm à 440 nm, la première composition comportant un filtre solaire non photostable, d'indice de photostabilité inférieur ou égal à 80 %.
- une deuxième composition, photorévélable thermiquement stable, à appliquer sur la couche de base.

**12.** Ensemble selon la revendication 11, la composition photorévélable thermiquement stable comportant un agent photorévélable thermiquement stable.

**13.** Ensemble selon la revendication 12, l'agent photorévélable étant choisi parmi les diaryléthènes et les fulgides.

**14.** Ensemble selon l'une quelconque des revendications 11 à 13, la première composition étant non aqueuse et la composition photorévélable thermiquement stable étant aqueuse, ou inversement.

**15.** Ensemble selon l'une quelconque des revendications 11 à 14, comportant un irradiateur capable d'émettre sélectivement dans l'UV et/ou le visible.

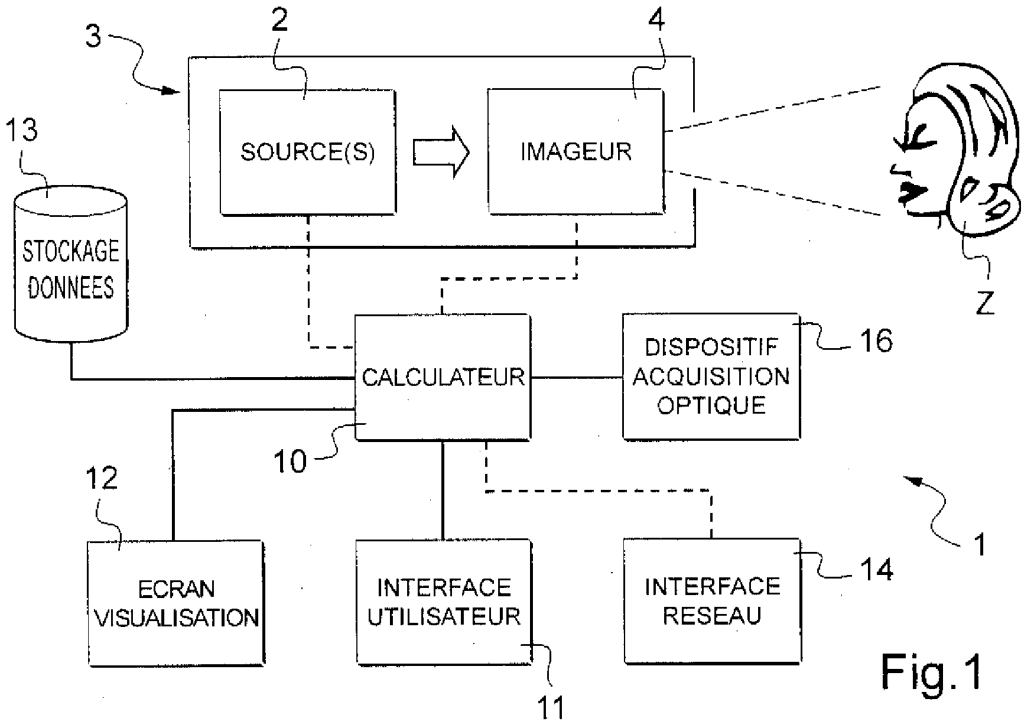


Fig.1

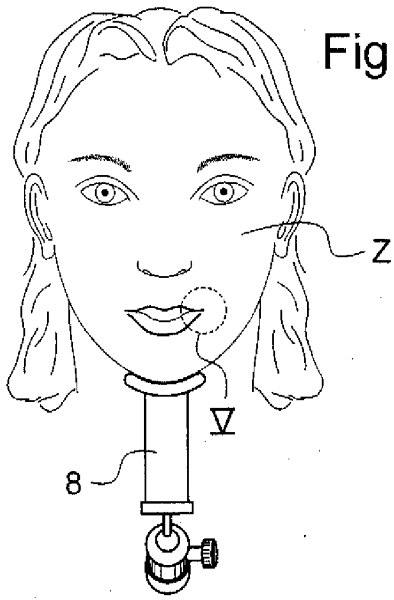


Fig.2

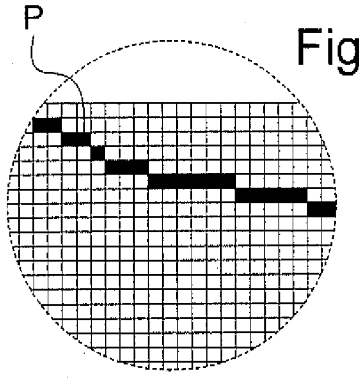


Fig.2a

Fig.3

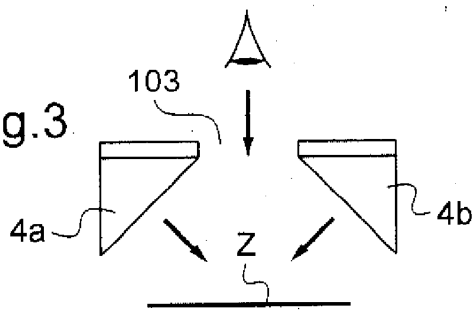


Fig.4

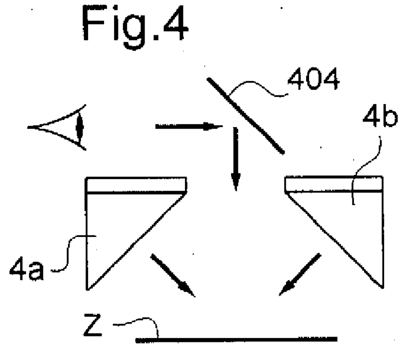

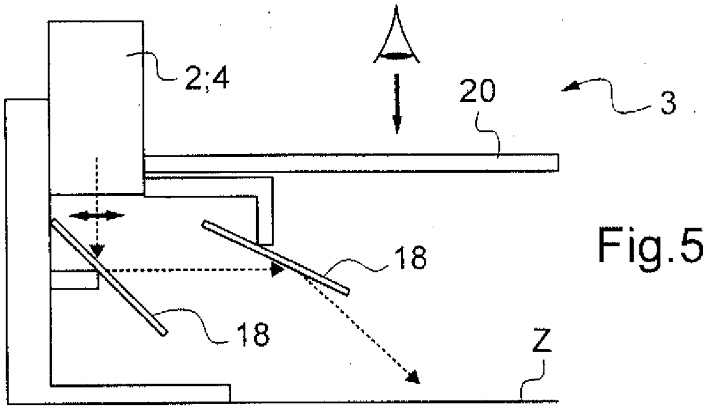


Fig.5

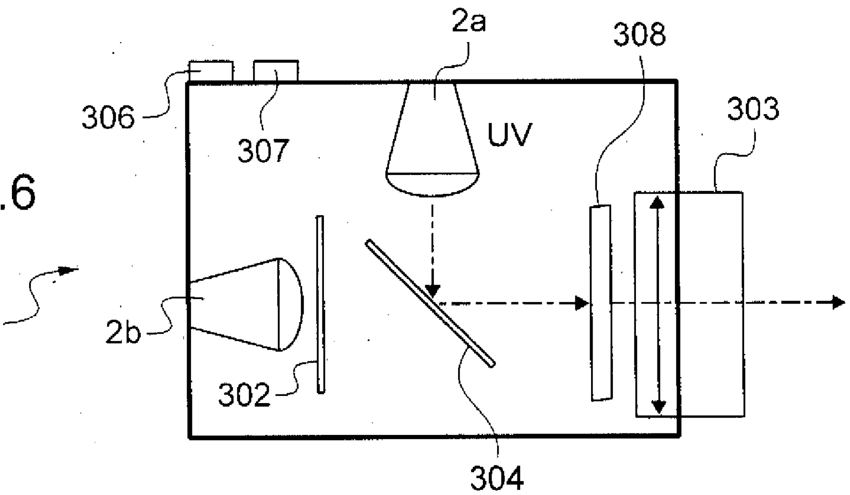


Fig.6

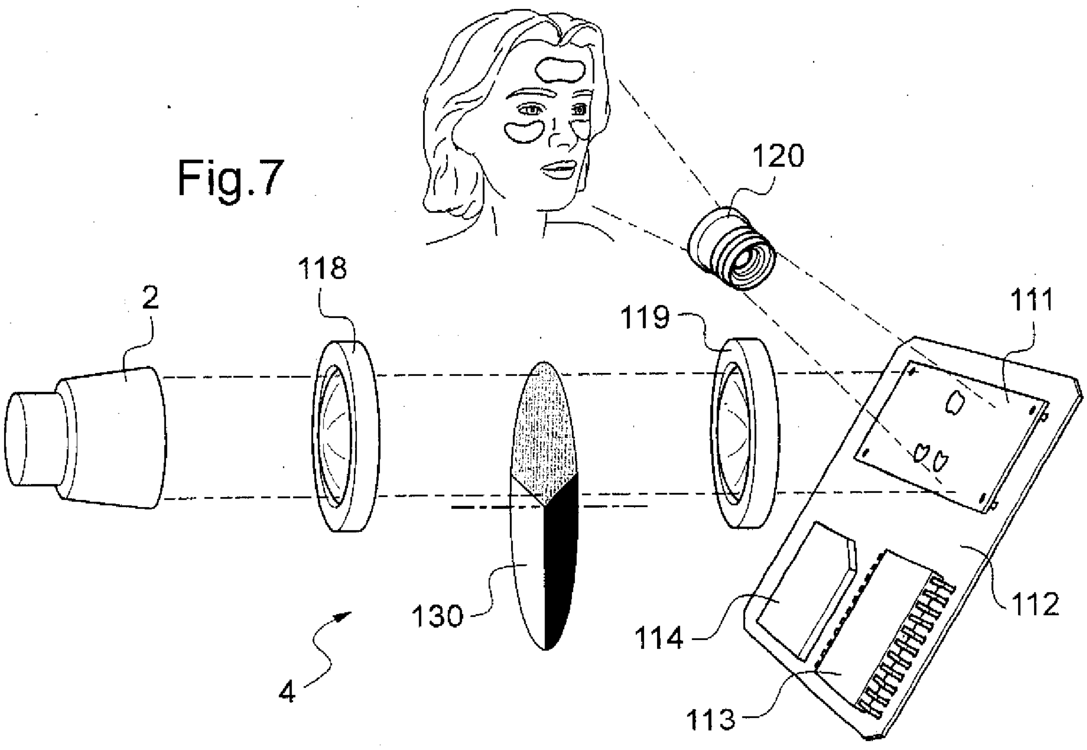


Fig.7

Fig.9

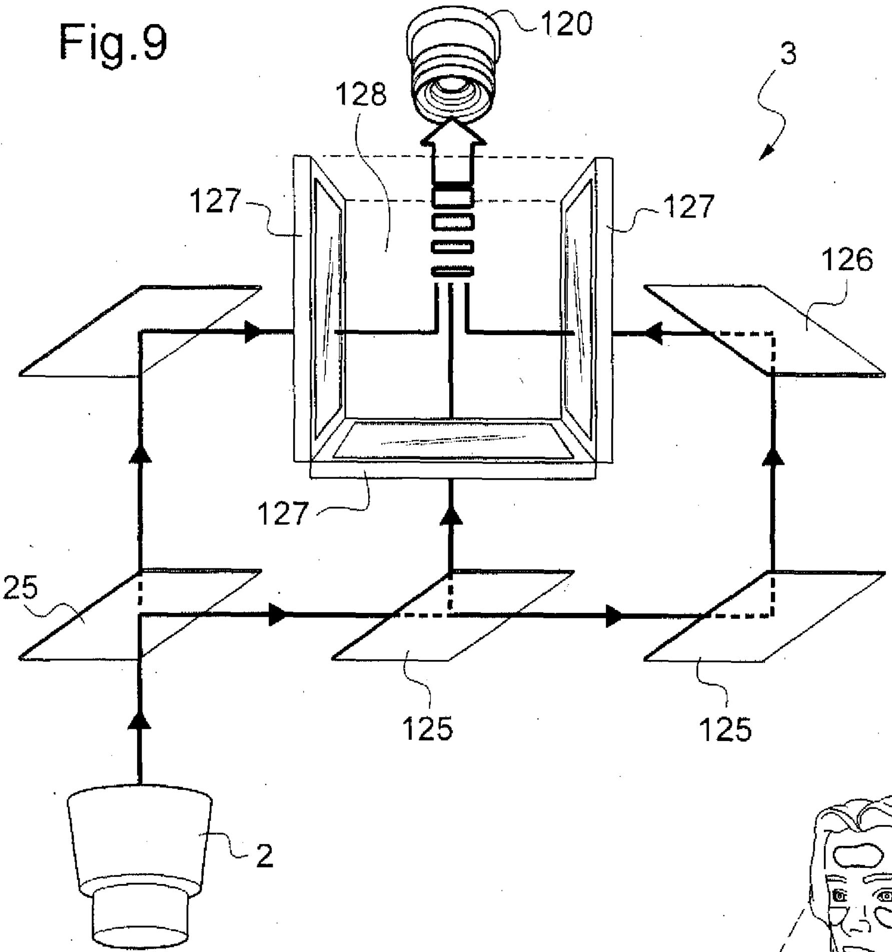


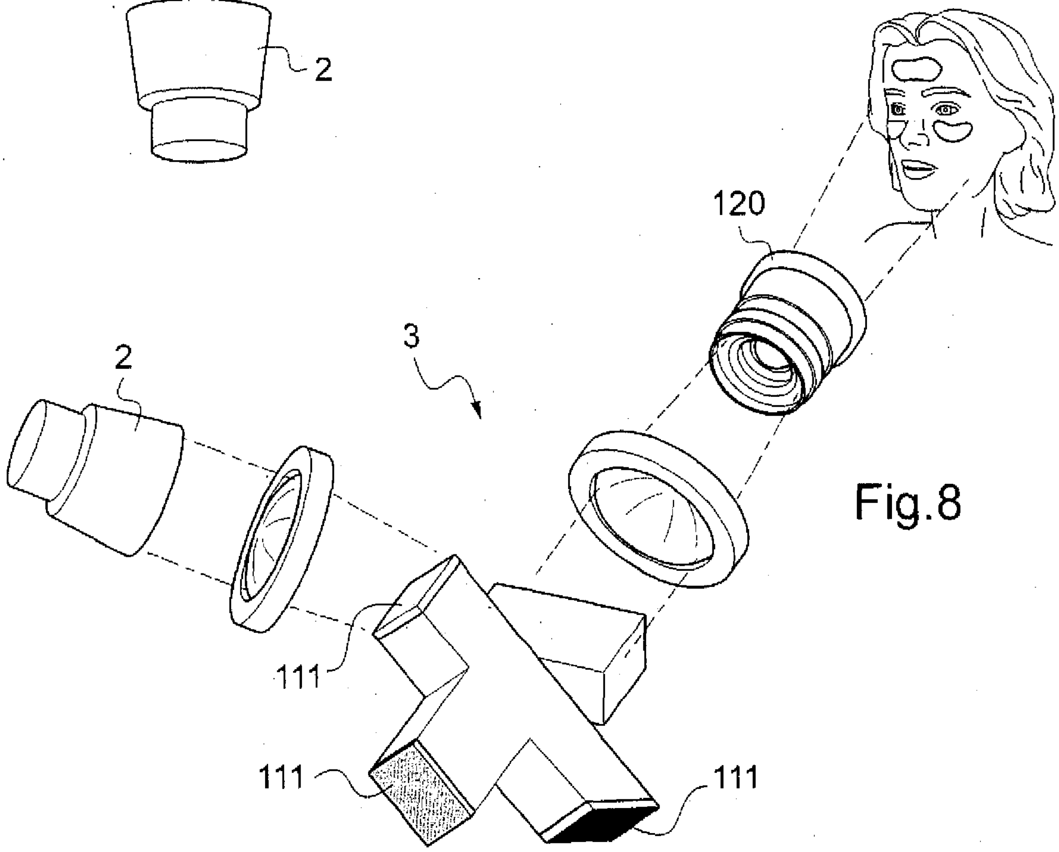


Fig.8

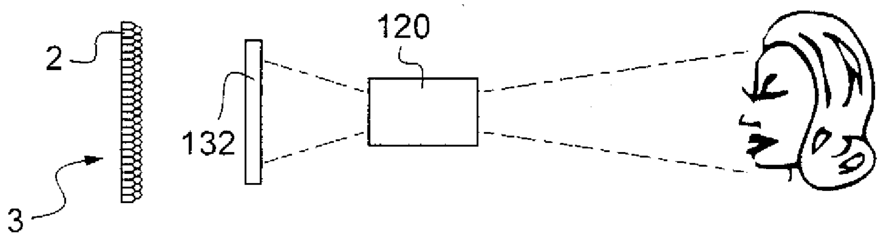


Fig.10

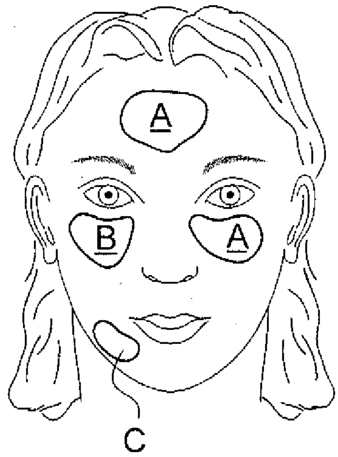


Fig.11

Fig.12

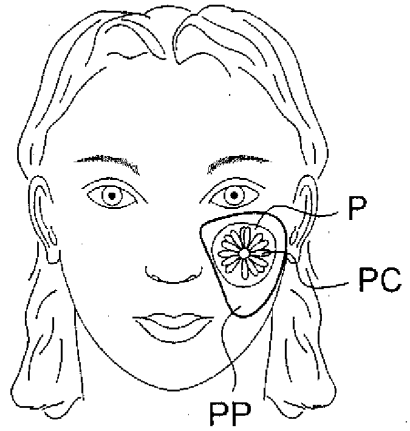


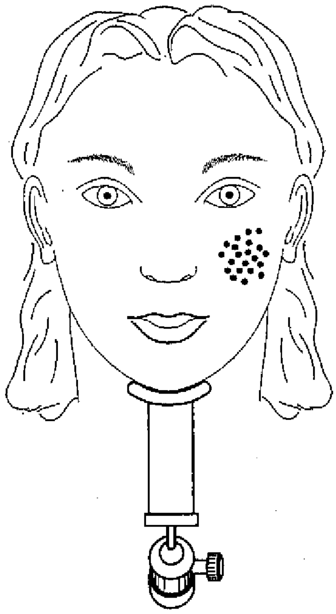

Fig.13

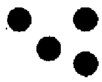

Fig.14A

Fig.14B

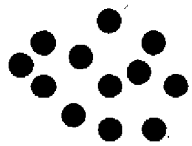

Fig.14C

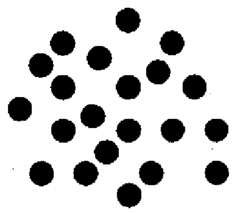

Fig.15A

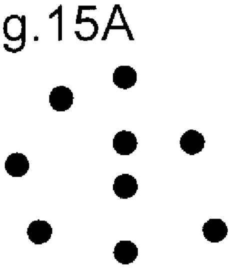

Fig.15B

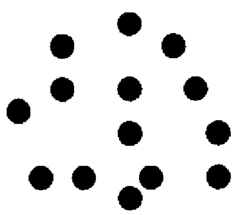

Fig.15C

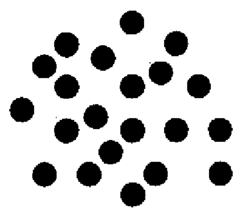

Fig.10A

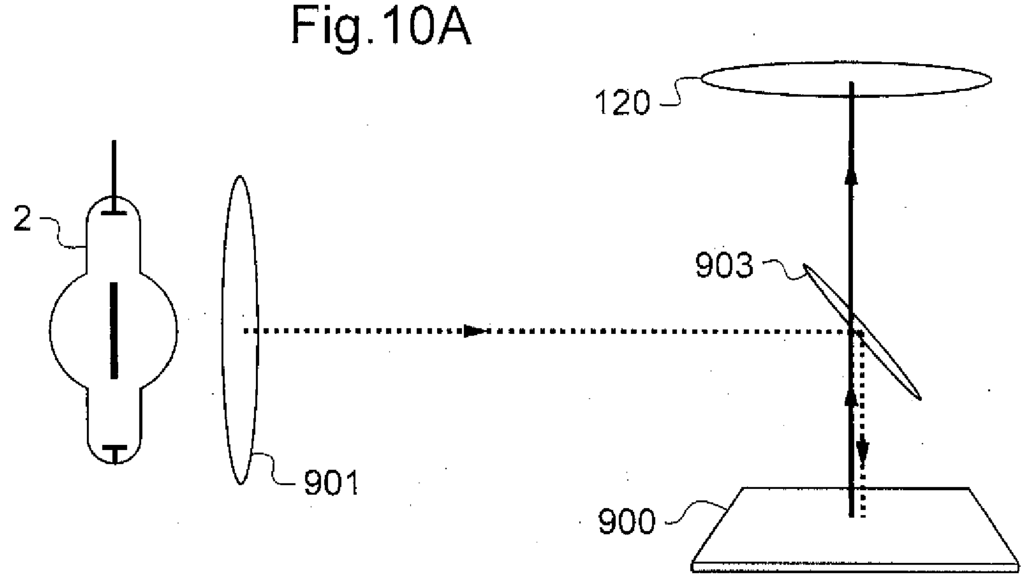


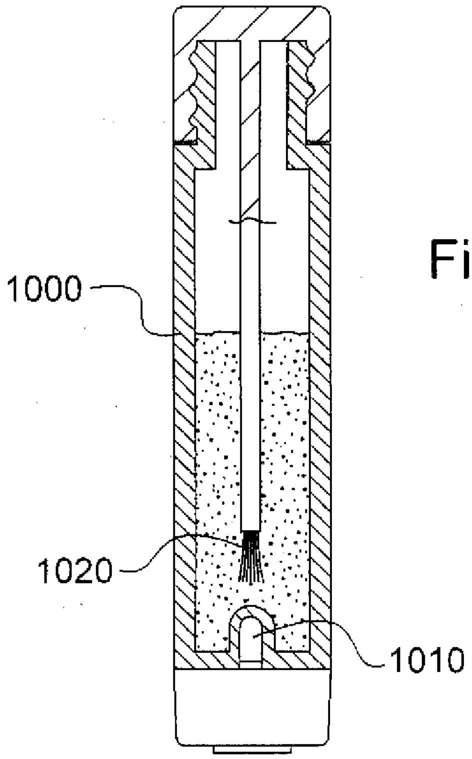


Fig.16

Europäisches Patentamt
European Patent Office
Office européen des brevets

# RAPPORT DE RECHERCHE EUROPEENNE

Numéro de la demande
EP 10 15 4374

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X<br><br>Y | EP 1 410 785 A (OREAL [FR])<br>21 avril 2004 (2004-04-21)<br>* alinéas [0018] - [0027], [0171] - [0177]; exemples 1-3 * | 1<br><br>1-15 | INV.<br>A61K8/02<br>A61K8/49<br>A61Q1/00<br>A61Q3/02<br>A61Q5/00 |
| X<br><br>Y | WO 02/03949 A (PROCTER & GAMBLE [US])<br>17 janvier 2002 (2002-01-17)<br>* revendication 9; exemples 1-11 * | 11-15<br><br>1-15 | |
| A | DOUGLAS WOLK: "There goes the sun- Which sunscreens work best?"<br>INTERNET CITATION<br>9 août 2005 (2005-08-09), pages 6-7, XP002552532<br>Extrait de l'Internet:<br>URL:http://www.slate.com/id/2124091/><br>[extrait le 2009-10-26]<br>* page 6 - page 7 * | 1-15 | |
| X<br><br>Y | FR 2 780 275 A (OREAL [FR])<br>31 décembre 1999 (1999-12-31)<br>* page 1 - page 2; revendications 3,20-23; exemple 1 * | 11,12<br><br>1-15 | DOMAINES TECHNIQUES RECHERCHES (IPC)<br>A61Q<br>A61K |
| Y,D | EP 0 938 887 A (OREAL [FR])<br>1 septembre 1999 (1999-09-01)<br>* le document en entier * | 1-15 | |
| Y | US 2008/089917 A1 (DUMOUSSEAUX CHRISTOPHE [JP] ET AL) 17 avril 2008 (2008-04-17)<br>* alinéas [0261] - [0312] * | 1-15 | |

Le présent rapport a été établi pour toutes les revendications

1

| Lieu de la recherche | Date d'achèvement de la recherche | Examinateur |
|---|---|---|
| Munich | 28 mai 2010 | Sala-Jung, Nathalie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 10 15 4374

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-05-2010

| Document brevet cité au rapport de recherche | | Date de publication | Membre(s) de la famille de brevet(s) | | | Date de publication |
|---|---|---|---|---|---|---|
| EP 1410785 | A | 21-04-2004 | AT | 350105 | T | 15-01-2007 |
| | | | DE | 60310846 | T2 | 11-10-2007 |
| | | | ES | 2280711 | T3 | 16-09-2007 |
| | | | JP | 4224384 | B2 | 12-02-2009 |
| | | | JP | 2004231635 | A | 19-08-2004 |
| WO 0203949 | A | 17-01-2002 | AU | 7159301 | A | 21-01-2002 |
| FR 2780275 | A | 31-12-1999 | BR | 9903099 | A | 06-02-2001 |
| | | | CA | 2275963 | A1 | 25-12-1999 |
| | | | DE | 69902973 | D1 | 24-10-2002 |
| | | | DE | 69902973 | T2 | 23-01-2003 |
| | | | EP | 0970689 | A2 | 12-01-2000 |
| | | | ES | 2184393 | T3 | 01-04-2003 |
| | | | JP | 3556125 | B2 | 18-08-2004 |
| | | | JP | 2000038329 | A | 08-02-2000 |
| | | | KR | 20000006356 | A | 25-01-2000 |
| | | | US | 6080415 | A | 27-06-2000 |
| EP 0938887 | A | 01-09-1999 | AT | 254442 | T | 15-12-2003 |
| | | | CA | 2254322 | A1 | 12-06-1999 |
| | | | DE | 69819866 | D1 | 24-12-2003 |
| | | | DE | 69819866 | T2 | 02-09-2004 |
| | | | ES | 2212241 | T3 | 16-07-2004 |
| | | | FR | 2772266 | A1 | 18-06-1999 |
| | | | JP | 3590731 | B2 | 17-11-2004 |
| | | | JP | 11236309 | A | 31-08-1999 |
| | | | US | 6123952 | A | 26-09-2000 |
| US 2008089917 | A1 | 17-04-2008 | EP | 1913933 | A1 | 23-04-2008 |
| | | | FR | 2907009 | A1 | 18-04-2008 |
| | | | JP | 2008094849 | A | 24-04-2008 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 938887 A
- US 20070038270 A1
- EP 0938887 A1
- WO 9304665 A
- EP 678292 A
- FR 2326405
- FR 2440933
- EP 0114607 A
- FR 2679771
- US 6225198 B
- US 5990479 A
- CA 1306954
- US 5456905 A
- EP 1247515 A
- EP 1572139 A
- FR 2910315
- WO 0125283 A
- EP 759812 A


### Littérature non-brevet citée dans la description


- **C P McCoy et al.** *J. Am. Chem. Soc.,* 2007, vol. 129, 9572
- *Applied Optics,* 01 Juin 2002, vol. 41 (6
- **Dabboussi B.O. et al.** (CdSe)ZnS core-shell quantum dots : synthesis and characterisation of a size series of highly luminescent nanocristallites. *Journal of phisical chemistry B,* 1997, vol. 101, 9463-9475
- **Peng, Xiaogang et al.** Epitaxial Growth of highly Luminescent CdSe/CdS core/shell nanocrystals with photostability and electronic accessibility. *Journal of the American Chemical Society,* vol. 119 (30), 7019-7029
- **Hermanson GT.** Bioconjugate Techniques. Academic Press, 1996